# EUROPEAN PATENT SPECIFICATION

(11) **EP 2 464 654 B1**
(45) Date of publication and mention of the grant of the patent: **08.10.2014**
(21) Application number: 10747930.5
(22) Date of filing: 09.08.2010
(51) Int. Cl.: C07K 1/00, A61L 27/28

(54) **THIOL PROTECTING GROUP**
THIOL-SCHUTZGRUPPE
GROUPEMENT PROTECTEUR THIOL

(30) Priority: 10.08.2009 GB 0913965; 10.08.2009 GB 0913967; 14.08.2009 GB 0914321
(43) Date of publication of application: 20.06.2012
(73) Proprietor: UCL Business PLC, London W1T 4TP (GB)
(72) Inventor: SMITH, Mark, London W1T 4TP (GB); CADDICK, Stephen, London W1T 4TP (GB); BAKER, James, London W1T 4TP (GB); CHUDASAMA, Vijay, London W1T 4TP (GB)
(74) Representative: Srinivasan, Ravi Chandran
(86) International application number: PCT/GB2010/001500
(87) International publication number: WO 2011/018612

(56) References cited:
- WO-A1-94/10156
- BE-A- 725 964
- DE-A1- 2 032 709
- GB-A- 1 544 686
- US-A- 2 861 093
- COONEY D A ET AL: "Inhibition of L asparagine synthetase by mucochloric and mucobromic acids" ENZYME, vol. 21, no. 6, 1976, pages 524-539, XP008126942
- LEBETKIN E H ET AL: "Disposition of 3-chloro-4-(dichloromethyl)-5-hydroxy-2(5H )-furanone (MX) in B6C3F1 mice and F344 rats." JOURNAL OF TOXICOLOGY AND ENVIRONMENTAL HEALTH PART A, vol. 65, no. 24, 2002, pages 2101-2118, XP002612569
- YONEYAMA K ET AL: "ANTAGONISTIC MECHANISM OF SULFHYDRYL COMPOUNDS ON CELLOCIDIN ACTIVITY" JOURNAL OF ANTIBIOTICS, vol. 31, no. 10, 1978, pages 1065-1066, XP002612570
- SMITH M E B ET AL: "Protein modification, bioconjugation, and disulfide bridging using bromomaleimides." JOURNAL OF THE AMERICAN CHEMICAL SOCIETY, vol. 132, no. 6, 17 February 2010 (2010-02-17), pages 1960-1965, XP002601596
- TEDALDI L M ET AL: "Bromomaleimides: new reagents for the selective and reversible modification of cysteine." CHEMICAL COMMUNICATIONS, no. 43, 21 November 2009 (2009-11-21), pages 6583-6585, XP002601597

## Description

### INTRODUCTION

Synthesis of complex organic molecules frequently involves carrying out chemical transformations on reagents that carry multiple chemically reactive functional groups. A key aspect of such processes is the need to effect the desired chemical transformation at a specific functional group, while suppressing unwanted side reactions involving other functional groups. Thus, the use of chemical protecting groups is often a vital tool in achieving satisfactory specificity when carrying out the synthetic steps needed to produce a great many industrially and therapeutically useful compounds.

The fundamental principle of chemical protection is conceptually straightforward: by introducing a chemical protecting group onto a particular functional group on a molecule its reactivity is blocked, thus enabling a reaction to be effected with specificity at another functional group on that molecule. Underlying this simple premise, though, is a manifold of diverse considerations. These include the need for introduction of a particular protecting group to reach near quantitative yield under conditions that do not affect the remainder of the molecule, the requirement for the protecting group to remain fully in tact during subsequent synthetic steps, while not inducing side-reactions or otherwise affecting the chemistry of the process, and the need for the protecting group to be susceptible to removal when no longer required, again under conditions that do not impact elsewhere on the molecule. Furthermore, in many synthetic methods, multiple protecting groups are employed simultaneously. In such methods, it is often crucial that one protecting group can be removed under conditions that do not affect any others (the so-called "orthogonal" protecting group strategy). In this context, it is not surprising that a very large number of protecting groups are known and used routinely in the art.

Thiol functional groups (-SH) are well known to be highly nucleophilic and particularly susceptible to oxidation processes. Accordingly, protection of thiol groups in a molecule can be of particular importance during synthetic procedures. For example, the -CH₂SH α-substituent of the proteinogenic amino acid cysteine presents unique synthetic problems in the context of peptide synthesis, since in its unprotected form the thiol group would interfere with many routine operations.

The most popular strategy for protecting thiol groups is probably direct alkylation (for example, with t-butyl, trityl or acetamidomethyl groups). Disulfide formation (for example, with a t-butylthiol group) and acyl derivitisation (for example, to form thioesters, thiocarbonates and thiocarbamates) are also sometimes used to shield the thiol group while chemical transformations are effected on other parts of a molecule.

There remains a need for new protecting groups suitable for incorporation into organic synthetic methods. Particular considerations underpinning the development of new thiol protection strategies include finding groups which react with thiols with sufficient specificity and which can be readily cleaved as required, using reagents to which other functional groups (protected or not) in the molecule are chemically inert.

The present invention is based on the surprising finding that a reagent having a 1,2-dicarbonyl ethene moiety carrying an electrophilic leaving group on its C=C double bond can be used to protect a thiol group in a thiol compound. Advantages of this strategy include the following.
- The reaction between the reagent and the thiol compound is very rapid and can be carried out substantially stoichiometrically.
- The reagent is selective to thiol functional groups over other functional groups that are often present in complex organic molecules (for example, hydroxyl groups, amine groups and carboxyl groups). It therefore allows specific protection of a thiol functional group.
- The thioether bond formed during the chemical protection step is readily reversible, and in particular can be cleaved in a controlled manner at a time chosen by the skilled worker.
- The thioether bond is cleaved by reagents that are capable of acting as a nucleophile in a conjugate addition reaction. This specific reactivity profile can be exploited in a range of synthetic strategies. For example, use of the thiol protection strategy of the present invention can be particularly advantageous when the protected molecule does not contain any other functional groups which are reactive to conjugate addition reaction nucleophiles, or where one or more further protecting groups are present which are chemically inert to such reagents.

The methodology of the present invention is also ideally suited to protection of disulfide compounds. That is because incorporation of a second electrophilic leaving group, such that the 1,2-dicarbonyl ethene moiety carries an electrophilic leaving group on both carbons of its C=C double bond, renders the reagent capable of reacting with both of the sulfur atoms comprised in the disulfide moiety, thus forming a heterocyclic structure that masks the disulfide functional group.

Cooney et al (1976) ENZYME 21: 524-539 describes the reversible attachment of mucobromic acids to sulfhydril funtions on an enzyme.

### SUMMARY OF THE INVENTION

The present invention provides the use of a compound comprising a moiety of formula (I) as a reagent for protecting a thiol group in a thiol compound during a multi-step synthesis procedure 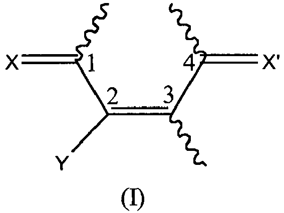 wherein X and X' are the same or different and each represents oxygen, sulfur or a group of formula =NR₁, in which R₁ is hydrogen, hydroxyl, C₁₋₆ alkyl or phenyl, and Y is an electrophilic leaving group.

The present invention also provides a process, which comprises:
(i) reacting a thiol compound with a compound comprising a moiety of formula (I) as herein defmed, thus producing a compound having a protected thiol group; and
(ii) subsequently deprotecting said protected thiol group;
   and wherein at least one step is carried out after step (i) and before step (ii) which comprises effecting a chemical transformation on a functional group carried by the thiol compound that is not the protected thiol group, under conditions to which the protected thiol group is chemically inert, but to which a thiol group would be chemically reactive.

The present invention also provides a use of a compound comprising a moiety of formula (III) as a reagent for protecting a disulfide group in a disulfide compound during a multi-step synthesis procedure 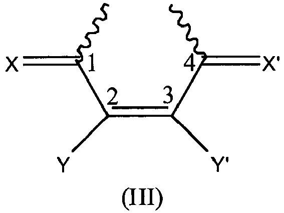 wherein X and X' are the same or different and each represents oxygen, sulfur or a group of formula =NR₁, in which R₁ is hydrogen, hydroxyl, C₁₋₆ alkyl or phenyl, and Y and Y' are the same or different and each represents an electrophilic leaving group.

Still further, the present invention provides a process, which comprises:
(i) reacting a disulfide group in a disulfide compound with a compound comprising a moiety of formula (III) of the present invention, thus producing a compound having a protected disulfide group; and
(ii) subsequently deprotecting said protected disulfide group;
and wherein at least one step is carried out after step (i) and before step (ii) which comprises effecting a chemical transformation on a functional group carried by the disulfide compound that is not the protected disulfide group, under conditions to which the protected disulfide group is chemically inert, but to which a disulfide group would be chemically reactive.

### BRIEF DESCRIPTION OF THE FIGURES

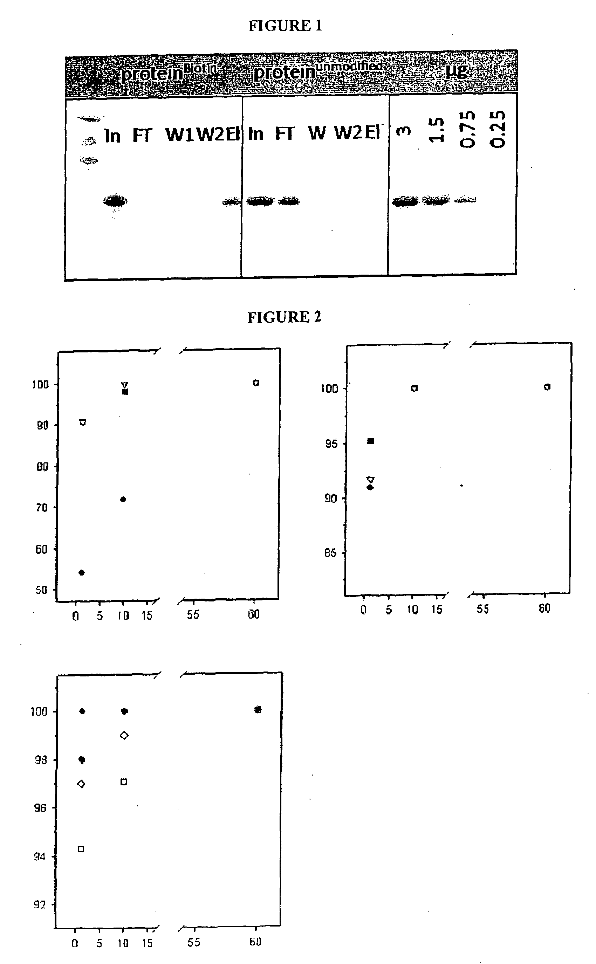

Figure 1 shows the results of the protocol described in Example 51 wherein protein / biotin-PEG-bromomaleimide adduct and unmodified model protein solutions (In) were added to neutravidin-coated agarose beads, centrifuged, the flow-through (FT) collected, the beads washed with PBS and both wash fractions collected (W1 and W2), protein released from the beads by incubation in PBS containing β-mercaptoethanol, the sample centrifuged and the eluant (E1) containing cleaved protein collected.
Figure 2 shows the generation of somatostatin-maleimide adducts from halomaleimides according to the protocol described in Example 65 as measured by LC-MS (y-axis = signal%; x-axis = time/min). Top left: Generation of somatostatin adduct from dichloromaleimide (circle), dibromomaleimide (square) and diiodomaleimide (triangle). Top right: Generation of somatostatin adduct from monobromomaleimide (circle), N-methylmonobromomaleimide (square) and N-methyldibromomaleimide (triangle). Bottom left: Generation of somatostatin adduct from N-fluorescein-dibromomaleimide (circle), N-biotin-dibromomaleimide (square), N-PEG 5000-dibromomaleimide (triangle), N-PEG-5000-dithiophenolmaleimide (diamond) and N-PEG 300-dibromomaleimide (oval).

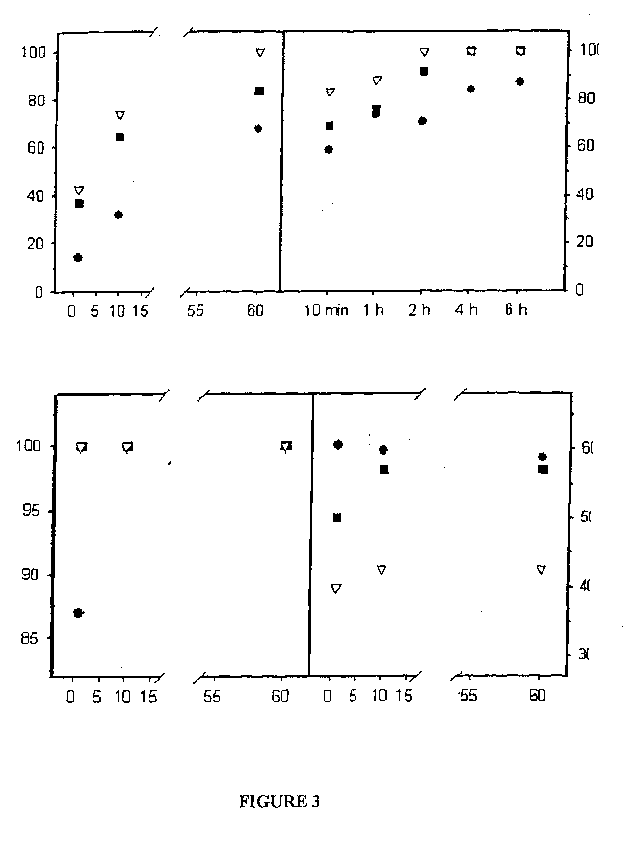

Figure 3 shows the generation of somatostatin-maleimide adducts from dithiomaleimides according to the protocol described in Example 65 as measured by LC-MS (y-axis = signal%; x-axis = time/min). Top left: Generation of somatostatin adduct from di-2-mercaptoethanolmaleimide at 1 eq. (circle), 5 eq. (square) and 10 eq. (triangle). Top right: Generation of somatostatin adduct from dicysteinemaleimide at 1 eq. (circle), 5 eq. (square) and 10 eq. (triangle). Bottom left: Generation of somatostatin adduct from dithiophenolmaleimide at 1 eq. (circle), 5 eq. (square) and 10 eq. (triangle). Bottom right: Generation of somatostatin adduct from di-2-mercaptopyridinemaleimide at 1 eq. (circle), 5 eq. (square) and 10 eq. (triangle).

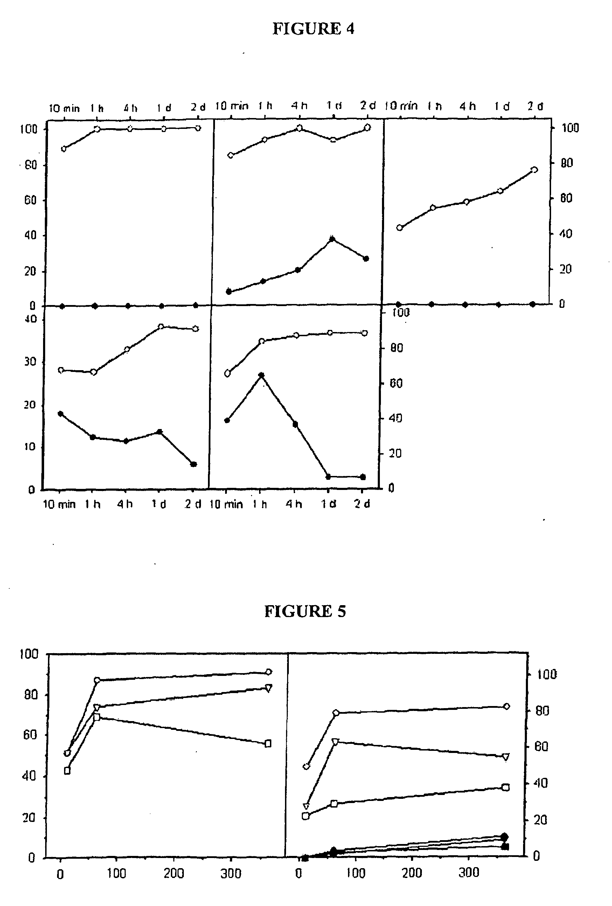

Figure 4 shows cleavage of maleimide bridged somatostatin with various reducing agents according to the protocol described in Example 65 as measured by LC-MS (y-axis = signal%; x-axis = time in minutes (min), hours (h) and days (d)). Top left: Total modified somatostatin-maleimide with DTT (hollow circle) and total amount of side products (filled circle). Top middle: Total modified somatostatin-maleimide with 2-mercaptoethanol (hollow circle) and total amount of side products (filled circle). Top right: Total modified somatostatin-maleimide with GSH (hollow circle) and total amount of side products (filled circle). Bottom left: Total modified somatostatin-maleimide with TCEP (hollow circle) and total amount of side products (filled circle). Bottom right: Total modified somatostatin-maleimide with 1,2-ethanedithiol (hollow circle) and total amount of side products (filled circle).
Figure 5 shows cleavage of maleimide bridged somatostatin with various amounts of DTT and 2-mercaptoethanol according to the protocol described in Example 65 as measured by LC-MS (y-axis = signal%; x-axis = time/min). Left: Regeneration of somatostatin by DTT at 50 eq. (hollow circle), 20 eq. (hollow triangle) and 10 eq. (hollow square). Right: Regeneration of somatostatin by 2-mercaptoethanol at 50 eq. (hollow circle), 20 eq. (hollow triangle) and 10 eq. (hollow square) and total amount of side products at 50 eq. (filled circle), 20 eq. (filled triangle) and 10 eq. (filled square).

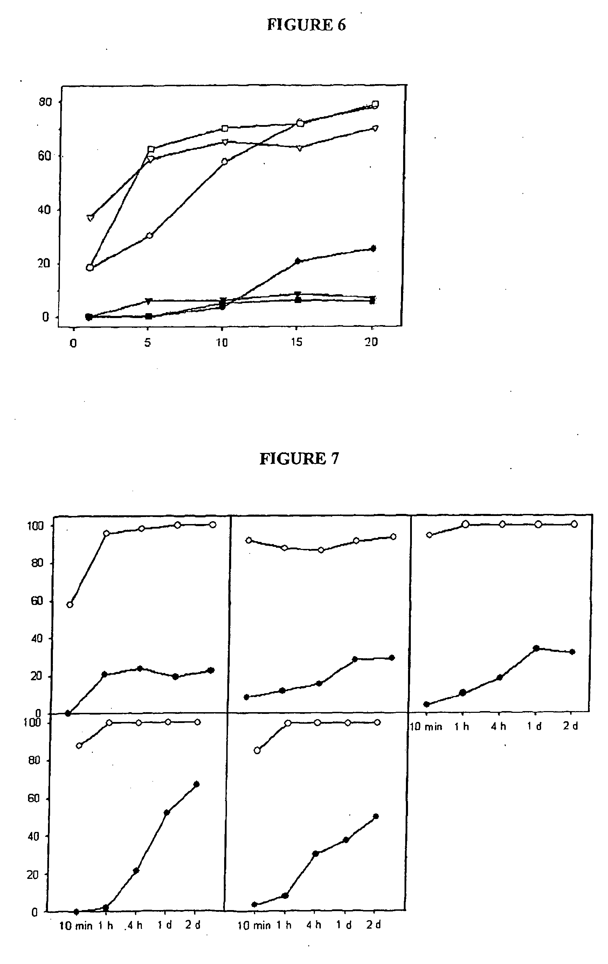

Figure 6 shows catalysed cleavage of bridged somatostatin according to the protocol described in Example 65 as measured by LC-MS (y-axis = signal%; x-axis = time/min). Shown on the Figure are regeneration of somatostatin by 2-mercaptoethanol (hollow circle), 2-mercaptoethanol with NaI (hollow square) and 2-mercaptoethanol with benzeneselenol (hollow triangle), as well as total side products when using 2-mercaptoethanol (filled circle), 2-mercaptoethanol with NaI (filled square) and 2-mercaptoethanol with benzeneselenol (filled triangle).
Figure 7 shows cleavage of N-functionalised maleimide bridged somatostatin by 2-mercaptoethanol according to the protocol described in Example 65 as measured by LC-MS (y-axis = signal%; x-axis = time in minutes (min), hours (h) and days (d)). Top left: cleavage of N-methylmaleimide somatostatin adduct to give somatostatin (hollow circle) and total side products (filled circle). Top middle: cleavage of N-biotin maleimide somatostatin adduct to give somatostatin (hollow circle) and total side products (filled circle). Top right: cleavage of N-fluorescein maleimide somatostatin adduct to give somatostatin (hollow circle) and total side products (filled circle). Bottom left: cleavage ofN-PEG 5000 maleimide somatostatin adduct to give somatostatin (hollow circle) and total side products (filled circle). Bottom middle: cleavage ofN-PEG 300 maleimide somatostatin adduct to give somatostatin (hollow circle) and total side products (filled circle).

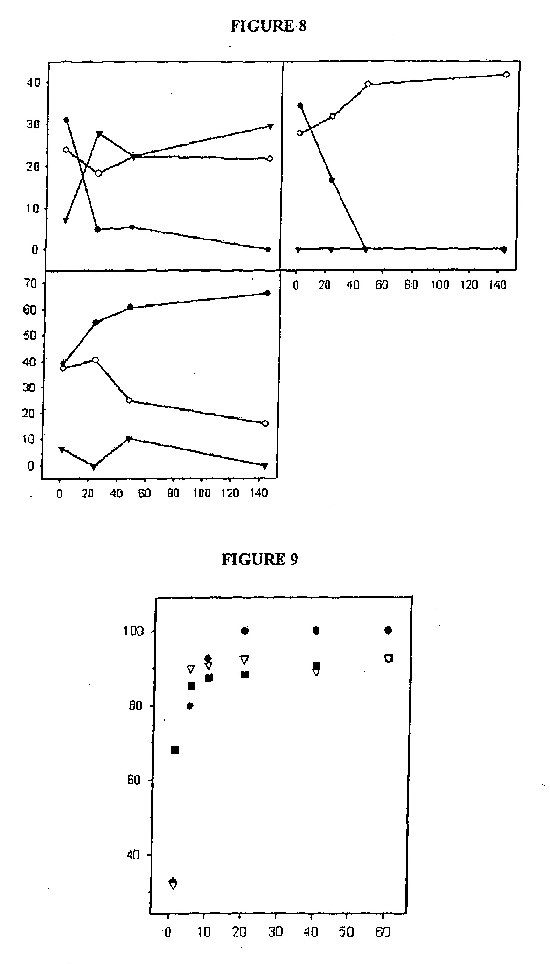

Figure 8 shows cleavage of the diaddition product of monobromomaleimide with somatostatin according to the protocol described in Example 65 as measured by LC-MS (y-axis = signal%; x-axis = time/hours). Top left: Somatostatin-maleimide (hollow circle), somatostatin-bis-maleimide (filled circle) and total side products (triangle) using 2-mercaptoethanol. Top right: Somatostatin-maleimide (hollow circle), somatostatin-bis-maleimide (filled circle) and total side products (triangle) using DTT. Bottom left: Somatostatin-maleimide (hollow circle), somatostatin-bis-maleimide (filled circle) and total side products (triangle) using TCEP.
Figure 9 shows comparable *in situ* bridging of somatostatin with various amounts of dithiomaleimides according to the protocol described in Example 65 as measured by LC-MS (y-axis = signal%; x-axis = time/min). The Figure shows generation of bridged somatostatin using TCEP initiator and thiophenol in a ratio of 3:5 (circle), selenol initiator with thiophenol in a ratio of 5:10 (square) and selenol initiator with 2-mercaptoethanol in a ratio of 10:20 (triangle).

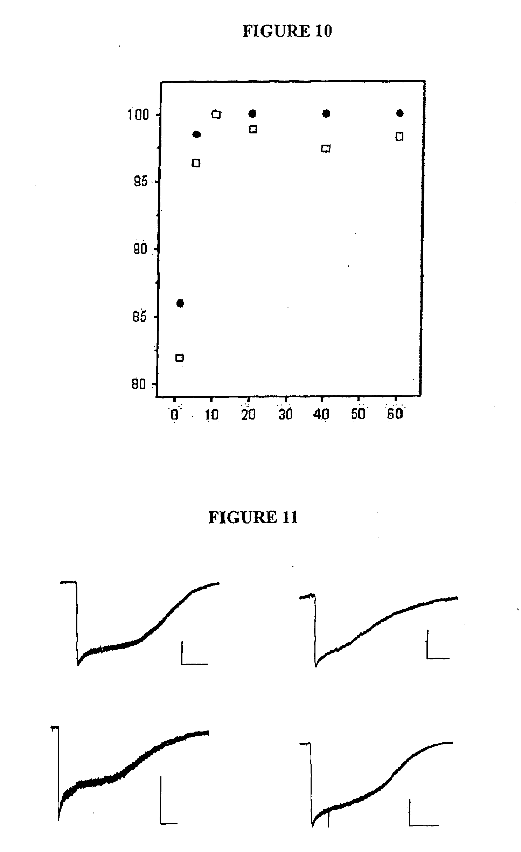

Figure 10 shows *in situ* PEGylation of somatostatin according to the protocol described in Example 65 as measured by LC-MS (y-axis = signal%; x-axis = time/min). The Figure shows generation of PEGylated somatostatin using 5 eq. N-PEG5000-dithiophenolmaleimide and 3 eq. TCEP (circle) and using 10 eq. N-PEG5000-dithiophenolmaleimide and 5 eq. benzeneselenol (square).
Figure 11 shows whole cell patch-clamp current recordings obtained in the patch clamp assay described in Example 65. The Figure shows representative traces recorded from the GIRK 1/2A cell line expressing SSTR2. The cells were clamped at -60mV and 20µM of somatostatin or its derivatives were applied for 20s. Top left: somatostatin (in the axes shown the vertical line represents 1000 pA and the horizontal line represents 20 ms). Top right: dibromomaleimide-bridged somatostatin (in the axes shown the vertical line represents 1000 pA and the horizontal line represents 20 ms). Bottom left: fluorescein dibromomaleimide-bridged somatostatin (in the axes shown the vertical line represents 1000 pA and the horizontal line represents 20 ms). Bottom right: PEGylated dibromomaleimide-bridged somatostatin (in the axes shown the vertical line represents 1000 pA and the horizontal line represents 20 ms).

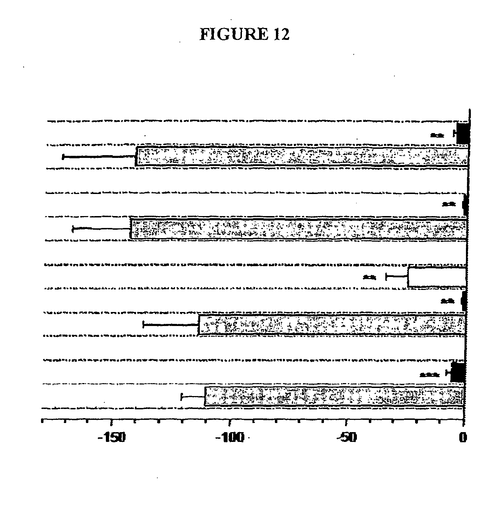

Figure 12 shows the amplitudes of the currents activated by somatostatin and its analogues in the patch clamp assay described in Example 65. The x-axis represents current amplitude in pA/pF. Top two bars are from fluorescein dibromomaleimide-bridged somatostatin (black bar is after pre-treatment of cell with Pertussis toxin for 24hr; grey bar is with no pre-treatment), next two bars are from PEGylated dibromomaleimide-bridged somatostatin (black bar is after pre-treatment of cell with Pertussis toxin for 24hr; grey bar is with no pre-treatment), next three bars are from dibromomaleimide-bridged somatostatin (white bar is after preincubation with the GIRK inhibitor TertiapinQ, 100nM for 5 minutes; black bar is after pre-treatment of cell with Pertussis toxin for 24hr; grey bar is with no pre-treatment) and bottom two bars are from somatostatin (black bar is after pre-treatment of cell with Pertussis toxin for 24hr; grey bar is with no pre-treatment).

### DETAILED DESCRIPTION OF THE INVENTION

As used herein, the terms "group" and "moiety" are used interchangeably.

As used herein, a "thiol compound" is a compound that contains at least one thiol group -SH.

As used herein, the term "electrophilic leaving group" means a substituent attached to a saturated or unsaturated carbon atom which can be replaced by a nucleophile following a nucleophilic attack at that carbon atom. Those of skill in the art are routinely able to select electrophilic leaving groups that would be suitable for locating on a particular compound and for reacting with a particular nucleophile.

As used herein, the term "nucleophile" means a functional group or compound which is capable of forming a chemical bond by donating an electron pair.

As used herein, the expression "protecting a thiol group" means introducing a protecting group onto a thiol group (-SH) carried on a thiol compound. The concept of chemical protection using protecting groups is of course well understood in the art of organic synthesis. Accordingly those of skill in the art would immediately recognise when a particular use of a reagent or a process involves "protecting a thiol group".

As used herein, the term "protecting group" means a group which has been introduced onto a functional group in a compound and which modifies the said functional group's chemical reactivity. Typically, the protecting group modifies the functional group's chemical reactivity in such a way that it renders the said functional group chemically inert to the reaction conditions used when a subsequent chemical transformation is effected on the said compound. A "thiol protecting group" is accordingly a protecting group which has been introduced onto the thiol group in a compound.

As those of skill in the art would immediately recognise, a protecting group is introduced onto a functional group in a compound through the reaction between the (unprotected) functional group and a protecting group precursor. The unprotected thiol functional group carries a proton which typically departs when the protected compound is formed, i.e. a thiol bond -S- is formed between the compound and the protecting group. In the present invention, the protecting group precursor is the compound comprising a moiety of formula (I). When the thiol group on the thiol compound reacts with the compound comprising a moiety of formula (I), the thiol group attaches to one of the carbon atoms on the C=C double by displacing the electrophilic leaving group Y. The protecting group of the present invention is thus a group comprising a moiety of formula (I'):

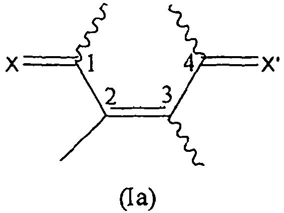

As used herein, a "protected functional group" means a functional group that is chemically protected via attachment to a protecting group. According to the present invention a "protected thiol group" is a thiol group that is attached to a group comprising a moiety of formula (I'). A "protected amine group" is a group obtainable by protecting an amine group (-NH₂). A "protected carboxyl group" is a group obtainable by protecting a carboxyl group (-COOH).

As used herein, "deprotecting" means removing a protecting group from a protected functional group. Thus, in,the present invention deprotecting the protected thiol group involves removing the group comprising a moiety of formula (I') from the protected thiol group.

As used herein, the term "orthogonal" in reference to two or more protecting groups means that there are reaction conditions under which each one of the protecting groups can be removed from the functional group to which it is attached, under which the other protecting groups are chemically inert. For example, where a first protecting group can be removed under basic conditions but not acidic conditions and a second protecting group can be removed under acidic conditions but not basic conditions, these first and second protecting groups constitute orthogonal protecting groups.

As used herein, the term "alkyl" includes both saturated straight chain and branched alkyl groups. Preferably, an alkyl group is a C₁₋₂₀ alkyl group, more preferably a C₁₋₁₅, more preferably still a C₁₋₁₂ alkyl group, more preferably still, a C₁₋₆ alkyl group, and most preferably a C₁₋₄ alkyl group. Particularly preferred alkyl groups include, for example, methyl, ethyl, propyl, isopropyl, butyl, isobutyl, tert-butyl, pentyl and hexyl. The term "alkylene" should be construed accordingly.

As used herein, the term "alkenyl" refers to a group containing one or more carbon-carbon double bonds, which may be branched or unbranched. Preferably the alkenyl group is a C₂₋₂₀ alkenyl group, more preferably a C₂₋₁₅ alkenyl group, more preferably still a C₂₋₁₂ alkenyl group, or preferably a C₂₋₆ alkenyl group, and most preferably a C₂₋₄ alkenyl group. The term "alkenylene" should be construed accordingly.

As used herein, the term "alkynyl" refers to a carbon chain containing one or more triple bonds, which may be branched or unbranched. Preferably the alkynyl group is a C₂₋₂₀ alkynyl group, more preferably a C₂₋₁₅ alkynyl group, more preferably still a C₂₋₁₂ alkynyl group, or preferably a C₂₋₆ alkynyl group and most preferably a C₂₋₄ alkynyl group. The term "alkynylene" should be construed accordingly.

Unless otherwise specified, an alkyl, alkenyl or alkynyl group is typically unsubstituted. However, where such a group is indicated to be unsubstituted or substituted, one or more hydrogen atoms are optionally replaced by halogen atom substituents. Preferably, a substituted alkyl, alkenyl or alkynyl group has from 1 to 10 substituents, more preferably 1 to 5 substituents, more preferably still 1, 2 or 3 substituents and most preferably 1 or 2 substituents, for example 1 substituent. Preferably, though, an alkyl, alkenyl or alkynyl group is unsubstituted.

In the moiety that is an alkyl, alkenyl or alkynyl group, in which (a) 0, 1 or 2 carbon atoms may be replaced by groups selected from C₆₋₁₀ arylene, 5- to 10-membered heteroarylene, C₃₋₇ carbocyclylene and 5- to 10-membered heterocyclylene groups, and (b) 0, 1 or 2 -CH₂- groups may be replaced by groups selected from -O-, -S-, -S-S-, -C(O)- and -N(C₁₋₆ alkyl)- groups, a total of 0, 1 or 2 of said carbon atoms and - CH₂- groups are preferably replaced, more preferably a total of 0 or 1. Most preferably, none of the carbon atoms or -CH₂- groups is replaced.

Preferred groups for replacing a -CH₂- group are -O-, -S- and -C(O)- groups. Preferred groups for replacing a carbon atom are phenylene, 5- to 6-membered heteroarylene, C₅₋₆ carbocyclylene and 5- to 6-membered heterocyclylene groups. As used herein, the reference to "0, 1 or 2 carbon atoms" means any terminal or non-terminal carbon atom in the alkyl, alkenyl or alkynyl chain, including any hydrogen - atoms attached to that carbon atom. As used herein, the reference to "0, 1 or 2 -CH₂-groups" refers to a group which does not correspond to a terminal carbon atom in the alkyl, alkenyl or alkynyl chain.

As used herein, a C₆₋₁₀ aryl group is a monocyclic or polycyclic 6- to 10-membered aromatic hydrocarbon ring system having from 6 to 10 carbon atoms. Phenyl is preferred. The term "arylene" should be construed accordingly.

As used herein, a 5- to 10- membered heteroaryl group is a monocyclic or polycyclic 5- to 10- membered aromatic ring system, such as a 5- or 6- membered ring, containing at least one heteroatom, for example 1, 2, 3 or 4 heteroatoms, selected from O, S and N. When the ring contains 4 heteroatoms these are preferably all nitrogen atoms. The term "heteroarylene" should be construed accordingly.

Examples of monocyclic heteroaryl groups include thienyl, furyl, pyrrolyl, imidazolyl, thiazolyl, isothiazolyl, pyrazolyl, oxazolyl, isoxazolyl, triazolyl, thiadiazolyl, oxadiazolyl, pyridinyl, pyridazinyl, pyrimidinyl, pyrazinyl, triazinyl and tetrazolyl groups.

Examples of polycyclic heteroaryl groups include benzothienyl, benzofuryl, benzimidazolyl, benzothiazolyl, benzisothiazolyl, benzoxazolyl, benzisoxazolyl, benztriazolyl, indolyl, isoindolyl and indazolyl groups. Preferred polycyclic groups include indolyl, isoindolyl, benzimidazolyl, indazolyl, benzofuryl, benzothienyl, benzoxazolyl, benzisoxazolyl, benzothiazolyl and benzisothiazolyl groups, more preferably benzimidazolyl, benzoxazolyl and benzothiazolyl, most preferably benzothiazolyl. However, monocyclic heteroaryl groups are preferred.

Preferably the heteroaryl group is a 5- to 6- membered heteroaryl group. Particularly preferred heteroaryl groups are thienyl, pyrrolyl, imidazolyl, thiazolyl, isothiazolyl, pyrazolyl, oxazolyl, isoxazolyl, triazolyl, pyridinyl, pyridazinyl, pyrimidinyl and pyrazinyl groups. More preferred groups are thienyl, pyridinyl, pyridazinyl, pyrimidinyl, pyrazinyl, pyrrolyl and triazinyl, most preferably pyridinyl.

As used herein, a 5- to 10- membered heterocyclyl group is a non-aromatic, saturated or unsaturated, monocyclic or polycyclic C₅₋₁₀ carbocyclic ring system in which one or more, for example 1, 2, 3 or 4, of the carbon atoms are replaced with a moiety selected from N, O, S, S(O) and S(O)₂. Preferably, the 5- to 10- membered heterocyclyl group is a 5- to 6- membered ring. The term "heterocyclyene" should be construed accordingly.

Examples of heterocyclyl groups include azetidinyl, oxetanyl, thietanyl, pyrrolidinyl, imidazolidinyl, oxazolidinyl, isoxazolidinyl, thiazolidinyl, isothiazolidinyl, tetrahydrofuranyl, tetrahydrothienyl, tetrahydropyranyl, tetrahydrothiopyranyl, dithiolanyl, dioxolanyl, pyrazolidinyl, piperidinyl, piperazinyl, hexahydropyrimidinyl, methylenedioxyphenyl, ethylenedioxyphenyl, thiomorpholinyl, S-oxo-thiomorpholinyl, S,S-dioxo-thiomorpholinyl, morpholinyl, 1,3-dioxolanyl, 1,4-dioxolanyl, trioxolanyl, trithianyl, imidazolinyl, pyranyl, pyrazolinyl, thioxolanyl, thioxothiazolidinyl, 1H-pyrazol-5-(4H)-onyl, 1,3,4-thiadiazol-2(3H)-thionyl, oxopyrrolidinyl, oxothiazolidinyl, oxopyrazolidinyl, succinimido and maleimido groups and moieties. Preferred heterocyclyl groups are pyrrolidinyl, imidazolidinyl, oxazolidinyl, isoxazolidinyl, thiazolidinyl, isothiazolidinyl, tetrahydrofuranyl, tetrahydrothienyl, tetrahydropyranyl, tetrahydrothiopyranyl, dithiolanyl, dioxolanyl, pyrazolidinyl, piperidinyl, piperazinyl, hexahydropyrimidinyl, thiomorpholinyl and morpholinyl groups and moieties. More preferred heterocyclyl groups are tetrahydropyranyl, tetrahydrothiopyranyl, thiomorpholinyl, tetrahydrofuranyl, tetrahydrothienyl, piperidinyl, morpholinyl and pyrrolidinyl groups.

For the avoidance of doubt, although the above definitions of heteroaryl and heterocyclyl groups refer to an "N" moiety which can be present in the ring, as will be evident to a skilled chemist the N atom will be protonated (or will carry a substituent as defined below) if it is attached to each of the adjacent ring atoms via a single bond.

As used herein, a C₃₋₇ carbocyclyl group is a non-aromatic saturated or unsaturated hydrocarbon ring having from 3 to 7 carbon atoms. Preferably it is a saturated or mono-unsaturated hydrocarbon ring (i.e. a cycloalkyl moiety or a cycloalkenyl moiety) having from 3 to 7 carbon atoms, more preferably having from 5 to 6 carbon atoms. Examples include cyclopropyl, cyclobutyl, cyclopentyl and cyclohexyl and their mono-unsaturated variants. Particularly preferred carbocyclic groups are cyclopentyl and cyclohexyl. The term "carbocyclylene" should be construed accordingly.

Where specified, 0, 1 or 2 carbon atoms in a carbocyclylene or heterocyclylene group may be replaced by -C(O)- groups. As used herein, the "carbon atoms" being replaced are understood to include the hydrogen atoms to which they are attached. When 1 or 2 carbon atoms are replaced, preferably two such carbon atoms are replaced. Preferred such carbocyclyl groups include a benzoquinone group and preferred such heterocyclyl groups include succinimido and maleimido groups.

Unless otherwise specified, an aryl, heteroaryl, carbocyclyl or heterocyclyl group is typically unsubstituted. However, where such a group is indicated to be unsubstituted or substituted, one or more hydrogen atoms are optionally replaced by halogen atoms or C₁₋₆ alkyl, C₁₋₆ alkoxy, C₁₋₆ alkylthiol, -N(C₁₋₆ alkyl)(C₁₋₆ alkyl) or nitro groups. Preferably, a substituted aryl, heteroaryl, carbocyclyl or heterocyclyl group has from 1 to 4 substituents, more preferably 1 to 2 substituents and most preferably 1 substituent. Preferably a substituted aryl, heteroaryl, carbocyclyl or heterocyclyl group carries not more than 2 nitro substituents. Preferred substituents are halogen atoms and C₁₋₄ alkyl and C₁₋₄ alkoxy groups. Particularly preferred substituents are halogen atoms. Preferably, though, an aryl, heteroaryl, carbocyclyl or heterocyclyl group is unsubstituted.

As used herein, halogen atoms are typically F, Cl, Br or I atoms, preferably Br or Cl atoms, more preferably Br atoms.

As used herein, a C₁₋₆ alkoxy group is a C₁₋₆ alkyl (e.g. a C₁₋₄ alkyl) group which is attached to an oxygen atom.

As used herein, a C₁₋₆ alkylthiol group is a C₁₋₆ alkyl (e.g. a C₁₋₄ alkyl) group which is attached to a sulfur atom.

As used herein, a 5- to 10-membered heterocyclylthiol is a 5- to 10-membered (e.g., a 5- to 6-membered) heterocyclyl group which is attached to a sulfur atom.

As used herein, a C₆₋₁₀ arylthiol is a C₆₋₁₀ aryl (e.g., a phenyl) group which is attached to a sulfur atom.

As used herein, a C₃₋₇ carbocyclylthiol is a C₃₋₇ carbocyclyl (e.g., a C₅₋₆ carbocyclyl) group which is attached to a sulfur atom.

For the avoidance of doubt, a triflate group is a trifluoromethanesulfonate group, a tosylate group is a *p*-toluenesulfonate group and a mesylate group is a methanesulfonate group.

Preferably X and X' are the same or different and each represents oxygen, sulfur or a group of formula =NH. More preferably, X and X' are the same or different and each represents oxygen or sulfur. Preferably at least one of X and X' represents oxygen. Most preferably, X and X' are both oxygen.

Y is preferably a halogen atom or a triflate, tosylate, mesylate, N-hydroxysuccinimidyl, N-hydroxysulfosuccinimidyl, C₁₋₆ alkylthiol, 5- to 10-membered heterocyclylthiol, C₆₋₁₀ arylthiol, C₃₋₇ carbocyclylthiol, -OC(O)CH₃, -OC(O)CF₃, phenyloxy, -NRₓR_{y}R_{z}⁺ or -PRₓR_{y}R_{z}⁺ group. More preferably, Y is a halogen atom or a triflate, tosylate, mesylate, N-hydroxysuccinimidyl, N-hydroxysulfosuccinimidyl, C₁₋₆ alkylthiol, 5- to 10-membered heterocyclylthiol, C₆₋₁₀ arylthiol or C₃₋₇ carbocyclylthiol. More preferably still Y is a halogen atom or a C₁₋₆ alkylthiol, 5- to 10-membered heterocyclylthiol, C₆₋₁₀ arylthiol or C₃₋₇ carbocyclylthiol. Most preferably Y is a halogen atom, particularly a bromine atom.

Rₓ, Ry and R_{z} are each preferably selected from hydrogen atoms and C₁₋₆ alkyl groups. More preferably Rₓ, Ry and R_{z} are each preferably selected from hydrogen atoms and methyl and ethyl groups. Preferably, in a particular -NRₓR_{y}R_{z}⁺ or -PRₓR_{y}R_{z}⁺ group, Rₓ, Ry and R_{z} are the same.

It will be clear that the moiety of formula (I) represents the key reactive moiety which according to the present invention allows a thiol-containing compound to be chemically protected. In the moiety of formula (I) (and the moiety of formula (III), as described in detail elsewhere), the symbol 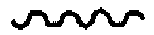 means a point of attachment to another group. It will be appreciated that the identity of the groups attached via these points of attachment is unimportant to the present invention. Those of skill in the art would readily understand that it would be possible to choose the groups attached via these points of attachment to suit a particular purpose, for example based on the specific identity of the thiol compound or disulfide compound to be protected.

Preferably, the compound comprising a moiety of formula (I) is a compound of formula (Ia) 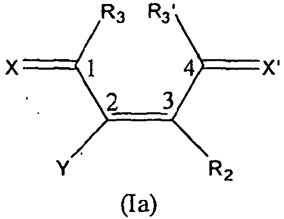 wherein:
- X and X' are the same or different and each represents oxygen, sulfur or a group of formula =NR₁, in which R₁ is hydrogen, hydroxyl, C₁₋₆ alkyl or phenyl;
- Y is an electrophilic leaving group;
- R₂ represents a hydrogen atom or a group IG;
- either:
   - R₃ and R₃' are the same or different and each represents a hydrogen atom or a group IG; or
   - R₃ and R₃' together form a group of formula -N(R_{33'}), wherein R_{33'} represents a hydrogen atom or a group IG; or
   - R₃ and R₃' together form a group of formula -N(R_{33'})-N(R_{33'})-, wherein
      each R_{33'} is the same or different and represents a hydrogen atom or a group IG; and
   - each group IG is the same or different and represents a moiety which is a C₁₋₂₀ alkyl group, a C₂₋₂₀ alkenyl group or a C₂₋₂₀ alkynyl group, which is unsubstituted or substituted by one or more halogen atoms and in which (a) 0, 1 or 2 carbon atoms are replaced by groups selected from C₆₋₁₀ arylene, 5- to 10-membered heteroarylene, C₃₋₇ carbocyclylene and 5- to 10-membered heterocyclylene groups, and (b) 0, 1 or 2 -CH₂- groups are replaced by groups selected from -O-, -S-, -S-S-, -C(O)-, -NH- and -N(C₁₋₆ alkyl)- groups, wherein:
      (i) said arylene, heteroarylene, carbocyclylene and heterocyclylene groups are unsubstituted or substituted by one or more substituents selected from halogen atoms and C₁₋₆ alkyl, C₁₋₆ alkoxy, C₁₋₆ alkylthiol, -N(C₁₋₆ alkyl)(C₁₋₆ alkyl) and nitro groups; and
      (ii) 0, 1 or 2 carbon atoms in said carbocyclylene and heterocyclylene groups are replaced by -C(O)- groups.

Preferably R₃ and R₃' are the same or different and each represents a hydrogen atom or a group IG or R₃ and R₃' together form a group of formula -N(R_{33'}), wherein R_{33'} represents a hydrogen atom or a group IG.

In one preferred embodiment, when R₂ is a group IG, preferably R₂ comprises a group selected from -O-, -S-, -NH- and -N(C₁₋₆ alkyl)- attached directly to the 3-position of the formula (Ia) (i.e., R₂ is a moiety in which a -CH₂- group adjacent to the 3-position is replaced by an -O-, -S-, -NH- or -N(C₁₋₆ alkyl)- group).

R₂ is preferably a hydrogen atom or a C₁₋₆ alkyl group. Most preferably, R₂ is a hydrogen atom.

When R₃ and R₃' are the same or different and each represents a hydrogen atom or a group IG, preferably at least one of R₃ and R₃' is a group IG. Preferably, both R₃ and R₃' represent a group IG.

When R₃, R₃', or R₃ and R₃' represents a group IG, the or each group IG is preferably a group of formula -Z(C₁₋₆ alkyl), wherein Z is a direct bond or a group -O-, -S-, -NH- or -N(C₁₋₆ alkyl). More preferably in this embodiment the or each group IG is a group of formula -Z(C₁₋₂ alkyl), wherein Z is a direct bond or a group -O-, -NH- or -N(C₁₋₆ alkyl). Most preferably in this embodiment, the or each group IG is a group of formula -Z(C₁₋₂ alkyl), wherein Z is a direct bond or a group -O- or -N(C₁₋₆ alkyl).

Preferably R₃ and R₃' together form a group of formula -N(R_{33'}).

R_{33'} preferably represents a hydrogen atom or a C₁₋₆ alkyl group, and most preferably R₃₃' represents a hydrogen atom or a methyl or ethyl group, for example a methyl or ethyl group.

IG preferably represents a moiety which is an unsubstituted C₁₋₆ alkyl group, C₂₋₆ alkenyl group or C₂₋₆ alkynyl group, in which (a) 0 or 1 carbon atom is replaced by a group selected from phenylene, 5- to 6-membered heteroarylene, C₅₋₆ carbocyclylene and 5- to 6-membered heterocyclylene groups, wherein said phenylene, heteroarylene, carbocyclylene and heterocyclylene groups are unsubstituted or substituted by one or two substituents selected from halogen atoms and C₁₋₄ alkyl and C₁₋₄ alkoxy groups, and (b) 0, 1 or 2 -CH₂- groups are replaced by groups selected from -O-, -S-, -C(O)-, -NH- and -N(C₁₋₆ alkyl)- groups.

More preferably, IG represents a moiety which is an unsubstituted C₁₋₆ alkyl group, in which (a) 0 or 1 carbon atom is replaced by a group selected from unsubstituted phenylene, 5- to 6-membered heteroarylene, C₅₋₆ carbocyclylene and 5- to 6-membered heterocyclylene groups, and (b) 0 or 1 -CH₂- groups are replaced by groups selected from -O-, -NH- and -N(C₁₋₆ alkyl)- groups.

More preferably still, IG represents an unsubstituted C₁₋₆ alkyl or phenyl group.

Most preferably, IG represents an unsubstituted C₁₋₆ alkyl group, for example a methyl or ethyl group.

Preferably, the group IG does not comprise any groups -NH- unless that group -NH-is located directly adjacent to a group -C(O)- or -C(S)-. More preferably, the group IG does not comprise any groups -NH- unless that group -NH- is located directly adjacent to a group -C(O)-.

>

Preferably the compound of formula (Ia) is a compound of formula (Ib) 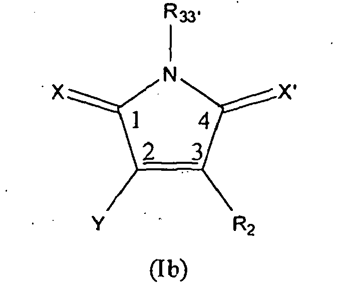 wherein X, X', Y, R₂ and R_{33'} are all as herein defined.

In the compound of formula (Ib) it is particularly preferred that
- X and X' each represent an oxygen atom;
- R_{33'} represents a hydrogen atom or a C₁₋₆ alkyl group;
- Y represents a halogen atom; and
- R₂ represents a hydrogen atom or a C₁₋₆ alkyl group.

In one embodiment, the present invention concerns the use of a compound containing the moiety of formula (I), as defined above, as a reagent for protecting a thiol group in a thiol compound. Typically, this use involves reacting the compound containing the moiety of formula (I) with the thiol compound. Reacting the compound containing the moiety of formula (I) with the thiol compound produces a compound in which the thiol group is protected with the protecting group of formula (I'), as defined above.

The compound containing the moiety of formula (I) can be reacted with the thiol compound according to procedures described in the standard literature and well known to those skilled in the art. Typical literature sources are *"*Advanced organic chemistry", 4th Edition (Wiley), J March, *"*Comprehensive Organic Transformation", 2nd Edition (Wiley), R.C. Larock , *"*Handbook of Heterocyclic Chemistry", 2nd Edition (Pergamon), A.R. Katritzky, review articles such as found in *"Synthesis", "Acc. Chem. Res.", "Chem. Rev",* or primary literature sources identified by standard literature searches online or from secondary sources such as *"Chemical Abstracts"* or *"Beilstein".*

More specifically, the use is a use of a compound comprising a moiety of formula (I) as a reagent for protecting a thiol group in a thiol compound during a multi-step synthesis procedure. Typically, the multi-step synthesis procedure comprises at least one synthesis step which comprises effecting a chemical transformation on a functional group carried by the thiol compound that is not the protected thiol group (for example, a functional group that is not a thiol group). Preferably, said at least one synthesis step is carried out under conditions to which the protected thiol group is chemically inert, but to which a thiol group would be chemically reactive. Typically, the multi-step synthesis procedure also comprises a step of deprotecting the protected thiol group.

In a further embodiment, the present invention relates to a process which involves reacting a thiol compound with a compound comprising a moiety of formula (I), thus producing a compound having a protected thiol group, and subsequently deprotecting said protected thiol group. This process therefore constitutes a means for selectively protecting a thiol group and then, later, removing the protecting group when it is no longer required.

One or more chemical reactions are effected on the thiol compound in between the steps (i) and (ii) of the process. As those of skill in the art would recognise, the nature of these steps will be determined by the overall synthetic procedure that is being carried out. At least one step is carried out after step (i) and before step (ii) which comprises effecting a chemical transformation on a functional group carried by the thiol compound that is not the protected thiol group (for example, a functional group that is not a thiol group). This at least one step is carried out under conditions to which the protected thiol group is chemically inert, but to which a thiol group would be chemically reactive.

In one embodiment of the process of the present invention the compound having a protected thiol group further comprises at least one additional protected functional group which is different from the protected thiol group. In this embodiment, preferably at least one additional protected functional group comprises a protecting group which is orthogonal to the protecting group on the protected thiol group.

The step of deprotecting the protected thiol group can be effected using routine methods for cleaving a thiol attached to an electron deficient alkene. Thus, preferably the deprotection is effected by incubating the product with a reagent that is capable of acting as a nucleophile in a conjugate addition reaction.

Examples of reagents that are well known to be capable of acting as a nucleophile in a conjugate addition reaction include phosphine compounds, phosphite compounds, thiols, selenols, amines and soft carbon nucleophilic compounds. Phosphine compounds and phosphite compounds both contain a trivalent phosphorous atom. In a phosphine, the phosphorous atom is attached to hydrogen or carbon atoms, while in a phosphite the phosphorous atom is attached to oxygen atoms (it being understood that the carbon atoms and oxygen atoms are themselves further attached to other groups in the respective compounds). Thiols are organic compounds containing a thiol group -SH. Selenols are organic compounds containing an -SeH group. Amines are compounds containing an amine functional group. Soft carbon nucleophiles are compounds which contain a soft nucleophilic carbon centre. Exemplary soft carbon nucleophiles are disclosed in US 5,414,074. Those of skill in the art would of course be able to select appropriate reagents that are capable of acting as a nucleophile in a conjugate addition reaction as a matter of routine, for example by routinely selecting a suitable reagent from amongst the exemplified classes of reagent herein described.

Presently preferred reagents for effecting the deprotection are phosphine compounds and thiols. A particularly preferred phosphine is tris(2-carboxyethyl)phosphine, which is commonly known as "TCEP" and is commonly used in the art to reduce disulfide bonds in compounds, for example, in proteins. Tris(2-carboxyethyl)phosphine can also be supplied in the form of a salt, such as its hydrochloride salt. Particularly preferred thiols are 1,2-ethanedithiol, glutathione, mercaptoethanol and dithiothreitol (i.e., HSCH₂CH(OH)CH(OH)CH₂SH, commonly known as DTT). For example, the reagent may be selected from tris(2-carboxyethyl)phosphine, glutathione, 2-mercaptoethanol and dithiothreitol.

For the avoidance of doubt, as used herein, the term "reagent that is capable of acting as a nucleophile in a conjugate addition reaction" means a reagent that is capable of reacting with an α,β-unsaturated moiety in a compound, and in particular a moiety of formula (IV) 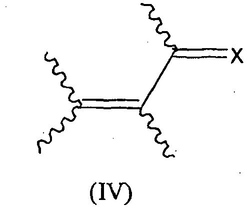 wherein X is as herein defined. Such reagents are sometimes known as "reagents that are capable of acting as a nucleophile in a Michael reaction". Clearly, the reagents are not limited to reagents which react through a nucleophilic carbon centre (e.g., soft carbon nucleophiles), but also include reagents which react through a nucleophilic non-carbon centre, such as the exemplary reagents that are described herein.

In the present invention, the thiol compound can be any compound containing a thiol group -SH. Typically, though, the thiol compound is an organic compound. As those of skill in the art would appreciate, the protecting group of the present invention can suitably be introduced onto a thiol group carried by any compound in which protecting group strategies are generally used (e.g., an intermediate in a multi-step synthesis procedure). Examples of thiol-containing compounds of particular interest in the biotechnological field are cysteine and peptides or proteins comprising at least one cysteine residue. Accordingly, in a preferred embodiment of the use and the process of the present invention, the thiol compound is cysteine or a peptide or protein comprising at least one cysteine residue. As those of skill in the art would appreciate, this embodiment is particularly useful when carrying out routine synthetic methods involving cysteine or cysteine residues, such as methods of peptide synthesis or methods in which peptides or proteins are subjected to chemical transformations (for example, to mimic post-translational modifications or to effect synthetic modifications with a view to undertaking proteomics studies or producing therapeutic proteins).

The present invention can be used, for example, to provide a compound of formula (II) wherein: 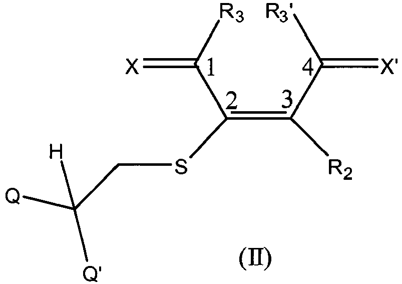
- X and X' are the same or different and each represents oxygen, sulfur or a group of formula =NR₁, in which R₁ is hydrogen, hydroxyl, C₁₋₆ alkyl or phenyl;
- Y is an electrophilic leaving group;
- R₂ represents a hydrogen atom or a group IG;
- either:
   - R₃ and R₃' are the same or different and each represents a hydrogen atom or a group IG; or
   - R₃ and R₃' together form a group of formula -N(R_{33'}), wherein R_{33'} represents a hydrogen atom or a group IG; or
   - R₃ and R₃' together form a group of formula -N(R_{33'})-N(R_{33'})-, wherein each R_{33'} is the same or different and represents a hydrogen atom or a group IG;
- each group IG is the same or different and represents a moiety which is a C₁₋₂₀ alkyl group, a C₂₋₂₀ alkenyl group or a C₂₋₂₀ alkynyl group, which is unsubstituted or substituted by one or more halogen atoms and in which (a) 0, 1 or 2 carbon atoms are replaced by groups selected from C₆₋₁₀ arylene, 5- to 10-membered heteroarylene, C₃₋₇ carbocyclylene and 5- to 10-membered heterocyclylene groups, and (b) 0, 1 or 2 -CH₂- groups are replaced by groups selected from -O-, -S-, -S-S-, -C(O)-, -NH- and -N(C₁₋₆ alkyl)- groups, wherein:
   (i) said arylene, heteroarylene, carbocyclylene and heterocyclylene groups are unsubstituted or substituted by one or more substituents selected from halogen atoms and C₁₋₆ alkyl, C₁₋₆ alkoxy, C₁₋₆ alkylthiol, -N(C₁₋₆ alkyl)(C₁₋₆ alkyl) and nitro groups; and
   (ii) 0, 1 or 2 carbon atoms in said carbocyclylene and heterocyclylene groups are replaced by -C(O)- groups;
- Q is a carboxyl group or a protected carboxyl group; and
- Q' is an amine group or a protected amine group;
with the proviso that said compound of formula (II) is not ¹⁴C-N-ethylmaleimide-S-cysteine or ¹⁴C-N-ethylmaleimide-S-N-acetyl-cysteine.

This compound constitutes a chemically protected cysteine molecule. It is useful in, for example, methods of peptide and protein synthesis. Methods of peptide and protein synthesis are very well known in the art and thus those of skill in the art would have no difficulty in applying the methodology of the present invention to carry out a method for producing a peptide or protein. One common reference textbook which discusses the various routine techniques routinely used in the art to synthesise peptides is "Amino Acid and Peptide Synthesis" (John Jones, Oxford Scientific Publications, 1992).

In a first embodiment, Q is a carboxyl group and Q' is an amine group, i.e. the protected cysteine is capable of reacting through either its carboxyl functional group or its amine functional group. This compound may be reacted either directly with a secondary compound or a further chemical protection step may be effected, for example to place a specific protecting group of interest on either the carboxyl functional group or the amine functional group.

In the second and third embodiments, Q is a protected carboxyl group and Q' is an amine group or Q is a carboxyl group and Q' is a protected amine group, respectively. Thus, in the second embodiment the molecule is capable of reacting only through its amine group while in the third embodiment the molecule is capable of reacting only through its carboxyl group.

In a fourth embodiment, Q is a protected carboxyl group and Q' is a protected amine group. This molecule is therefore protected at each of its three functional groups. It can be prepared for reaction by deprotecting one of these protected functional groups. In a particularly preferred embodiment, Q is a carboxyl group and Q' is a protected amine group. As those of skill in the art would appreciate, this molecule is particularly well suited to being used in routine methods of solid phase peptide synthesis, which typically involve successive cycles in which an N-protected amino acid is reacted at its carboxyl terminal with either a solid support (in the first cycle) or a growing peptide chain (in subsequent cycles), followed by N-deprotection and then further reaction with a new N-protected amino acid.

Techniques for solid phase synthesis of peptides and proteins are very well known in the art. A large number of solid supports activated with a range of reactive groups are commercially available, for example from Sigma Aldrich. Those of skill in the art would thus have no difficulty in applying the methodology of the present invention to carry out a process for producing a peptide or protein.

Protected carboxyl groups and protected amine groups are very well known in the art and could be routinely selected by those of skill in the art.

Exemplary protected carboxyl groups include the protected carboxyl groups described in "Protecting Group Chemistry" (Jeremy Robertson, Oxford University Press, 2003) and in "Amino Acid and Peptide Synthesis" (John Jones, Oxford Scientific Publications, 1992). Particularly preferred protected carboxyl groups are -COOQ, where Q is selected from C₁₋₆ alkyl, phenyl and benzyl groups.

Exemplary protected amine groups include the protected amine groups described in "Protecting Group Chemistry" (Jeremy Robertson, Oxford University Press, 2003) and in "Amino Acid and Peptide Synthesis" (John Jones, Oxford Scientific Publications, 1992). Particularly preferred protected amine groups are trityl (triphenylmethyl) and 2-nitrophenylsulphenyl groups and groups of formula -NHC(O)OQ', wherein Q' is selected from C₁₋₆ alkyl, phenyl, benzyl, 2-(4-biphenyl)-isopropyl, 9-fluorenylmethyl. Particularly preferred protected amine groups are those of formula -NHC(O)OQ', wherein Q' is t-butyl or 9-fluorenylmethyl (i.e., "BOC-protected amine" or "FMoc-protected amine").

Preferably the compound of formula (II) is not C-N-ethylmaleimide-S-cysteine or C-N-ethylmaleimide-S-N-acetyl-cysteine.

In a further aspect, the present invention relates to the use of a compound of formula comprising a moiety of formula (III) as a reagent for protecting a disulfide group in a disulfide compound.

A disulfide group is a divalent group of formula -S-S-. The internal S-S bond in a disulfide is often the weakest bond in an organic molecule. It is susceptible to cleavage under reductive conditions to yield two -SH moieties. Thus, where the disulfide compound is alicyclic, reduction of the disulfide leads to cleavage of the disulfide compound into two thiol-containing molecules. Where the disulfide disulfide is part of a heterocycle (e.g., where it has formed through condensation of two thiol groups present in a single molecule), the reduction of the disulfide bond leads to ring-opening. In both cases, highly nucleophilic thiol groups are generated. Clearly, therefore, the cleavage of disulfide bonds in a molecule can potentially have important consequences in the context of an organic synthesis procedure. Thus, where chemical transformations are carried out using reaction conditions to which a disulfide group may be reactive it may be desirable to chemically protect the disulfide group to prevent undesirable side reactions from occurring.

Typically, the disulfide group in the disulfide compound forms part of a heterocyclic ring (i.e., it has formed through condensation of two thiol groups present in a single molecule).

Disulfide bonds are commonly found in proteins via coupling of the thiol groups in two nearby cysteine residues and as such they often play an important role in the folding and stability of proteins. Thus, in a preferred embodiment the disulfide compound is a peptide or protein containing a disulfide group.

In the compound of formula (III), the groups X, X' and Y are preferably the same groups as those defined above. The group Y' is a second electrophilic leaving group. Preferred groups Y' correspond to the preferred groups Y as defined above. In the compound of formula (III), Y and Y' can be the same or different. Preferably, though, Y and Y' are the same.

Preferably the compound comprising a moiety of formula (III) is a compound of formula (IIIa) 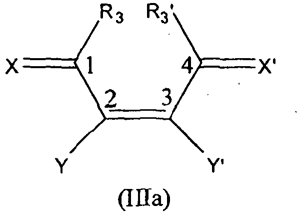 wherein X, X', Y, R₃ and R_{3'} are all as defined above and Y' is a further group Y, each group Y being the same or different.

Typically, this use involves reacting the compound containing the moiety of formula (III) with the disulfide compound. Reacting the compound containing the moiety of formula (III) with the disulfide compound involves cleaving the S-S bond of the disulfide group in the disulfide compound, with one of the two sulfur atoms of the disulfide group then displacing the group Y and the other of the two sulfur atoms of the disulfide group displacing the group Y'.

The compound containing the moiety of formula (III) can be reacted with the disulfide compound according to procedures described in the standard literature and well known to those skilled in the art. Typical literature sources are *"*Advanced organic chemistry", 4th Edition (Wiley), J March, *"*Comprehensive Organic Transformation", 2nd Edition (Wiley), R.C. Larock , *"*Handbook of Heterocyclic Chemistry", 2nd Edition (Pergamon), A.R. Katritzky, review articles such as found in *"Synthesis", "Acc. Chem. Res.", "Chem. Rev",* or primary literature sources identified by standard literature searches online or from secondary sources such as *"Chemical Abstracts"* or *"Beilstein".* Preferably, the reaction is effected by reducing the disulfide group to form two separate thiol groups -SH and reacting these thiol groups with the moiety of formula (III). Reduction of a disulfide group can be carried out using routine methods, for example, by reaction with a reducing agent such as a phosphine, a thiol or a hydride agent. Preferred reducing agents are tris(2-carboxyethyl)phosphine, glutathione, mercaptoethanol and dithiothreitol.

More specifically, the use is a use of a compound comprising a moiety of formula (III) as a reagent for protecting a disulfide group in a disulfide compound during a multi-step synthesis procedure. Typically, the multi-step synthesis procedure comprises at least one synthesis step which comprises effecting a chemical transformation on a functional group carried by the disulfide compound that is not a disulfide group. Preferably, said at least one synthesis step is carried out under conditions to which the protected disulfide group is chemically inert, but to which a disulfide group would be chemically reactive. Typically, the multi-step synthesis procedure also comprises a step of deprotecting the protected disulfide group (i.e., reforming the disulfide bond).

In a further embodiment, the present invention relates to a process which involves reacting a disulfide group in a disulfide compound with a compound comprising a moiety of formula (III), thus producing a compound having a protected disulfide group, and subsequently deprotecting said protected disulfide group. This process therefore constitutes a means for selectively protecting a disulfide group and then, later, removing the protecting group when it is no longer required.

Typically, the step of reacting the disulfide group in a disulfide compound with the compound comprising a moiety of formula (III) comprises (a) reducing the disulfide group to form two separate thiol groups -SH and (b) reacting these thiol groups with the moiety of formula (III). Reduction of the disulfide group can be carried out using routine methods, for example, by reaction with a reducing agent such as a phosphine, a thiol or a hydride agent. Preferred reducing agents are tris(2-carboxyethyl)phosphine, glutathione, 2-mercaptoethanol and dithiothreitol. Another preferred reducing agent is 1,2-ethanedithiol. Clearly, the disulfide group may be reduced in a first step, followed by a separate reaction with the moiety of formula (III). Alternatively, reduction of the disulfide group and the reaction with the moiety of formula (III) may be carried out in a single reaction step, i.e. with both the reducing agent and the moiety of formula (III) present simultaneously.

One or more chemical reactions are effected on the compound in between the steps (i) and (ii) of the process. As those of skill in the art would recognise, the nature of these steps will be determined by the overall synthetic procedure that is being carried out. At least one step is carried out after step (i) and before step (ii) which comprises effecting a chemical transformation on a functional group carried by the compound that is not the protected disulfide group (for example, a functional group that is not a disulfide group). This at least one step is carried out under conditions to which the protected disulfide group is chemically inert, but to which a disulfide group would be chemically reactive.

In one embodiment of the process of the present invention the compound having a protected disulfide group further comprises at least one additional protected functional group which is different from the protected disulfide group. In this embodiment, preferably at least one additional protected functional group comprises a protecting group which is orthogonal to the protecting group on the protected disulfide group.

The step of deprotecting the protected disulfide group can be effected using routine methods for cleaving a thiol attached to an electron deficient alkene. Thus, preferably the deprotection is effected by incubating the product with a reagent that is capable of acting as a nucleophile in a conjugate addition reaction. Preferred reagents for effecting the disulfide deprotection thus correspond to those set out above for effecting thiol deprotection.

Preferably the step of deprotecting the protected disulfide group involves cleaving the thiol bonds at the carbon-carbon double bond and also oxidising the two thiol groups thus formed so that they form a disulfide group. Suitable reagents for oxidising thiol groups to form a disulfide group are well known in the art. Exemplary oxidising reagents include iodine (I₂) and hydrogen peroxide, with iodine being preferred.

### EXAMPLES

The following Examples illustrate the scientific principles underlying the present invention. Protection and deprotection of a wide range of thiol and disulfide compounds by a wide range of protecting groups has been demonstrated, evidencing the broad applicability of the present invention.

¹H and ¹³C NMR spectra were recorded at room temperature on a Bruker Avance 500 instrument operating at a frequency of 500 MHz for ¹H and 125 MHz for ¹³C. ¹H NMR spectra were referenced to the CDCl₃ (7.26 ppm) signal. ¹³C NMR spectra were referenced to the CDCl₃ (77.67 ppm) signal.
Infra-red spectra were run on a PerkinElmer Spectrum 100 FT-IR spectrometer operating in ATR mode with frequencies given in reciprocal centimetres (cm⁻¹). Mass spectra and high resolution mass data were recorded on a VG70-SE mass spectrometer (EI mode and CI mode).
Melting points (m.p.) were taken on a Gallenkamp heating block and are uncorrected. Optical rotation measurements were carried out using a PerkinElmer 343 polarimeter with a cell length of 10 cm.

### Abbreviations

- Boc: *Tert*-butyloxycarbonyl group.
- Cys: Cysteine
- Mal: Maleimide
- DMF: Dimethylformamide
- TCEP: (tris(2-carboxyethyl)phosphine)
- LC-ESI-MS: Liquid chromatography electron spray ionisation mass spectroscopy

### Reference Example 1: Preparation of bromomaleimide (compound 1)

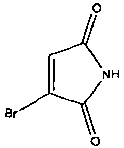

To maleimide (2.00 g, 0.02 mol) in chloroform (15 mL) was added bromine (1.16 mL, 0.02 mol) in chloroform (15 mL) dropwise. The reaction mixture was refluxed for 2 hours and left to cool to room temperature over 1 hour. Solid yellow precipitate was filtered off and washed with cold chloroform (2 x 50 mL) to afford white crystals of crude 2,3-dibromosuccinimide (4.09 g, 0.016 mol). The crude succinimide was dissolved in tetrahydrofuran (50 mL) and triethylamine (2.4 mL, 0.017 mol) in tetrahydrofuran (10 mL) was added over 5 minutes at 0 °C. The reaction mixture was allowed to warm to room temperature and stirred for 48 hours. The solid was filtered off and washed with tetrahydrofuran (50 mL) to afford a pale yellow powder (2.14 g, 0.012 mol) in 59 % yield.

¹H NMR (500MHz, CDCl₃): δ = 7.67 (br s, 1H, NH), 6.89 (s, 1H, C=CH); ¹³C NMR (125MHz, CDCl₃): δ = 173.8 (C=O), 170.5 (C=O), 136.9 (-(Br)C=C-), 135.4 (-C=CH-); IR (solid, cm⁻¹): 3235 (s), 1709 (s); MS (CI+) *m*/*z*, (%): 178 (⁸¹M+, 32), 176 (⁷⁹M+, 32), 125 (25), 86 (100); Mass calculated for C₄H₃O₂N⁷⁹Br: 175.93472. Found: 175.93493; m.p. 148 - 151°C.

### Reference Example 2: Preparation of N-methylbromomaleimide (compound 2)

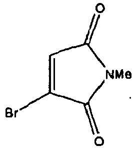

To N-methyl maleimide (0.5 g, 4.5 mmol) in methanol (22.5 mL) was added bromine (0.52 mL, 10 mmol) dropwise. The reaction mixture was stirred at room temperature for 24 hours. Solvent was removed *in vacuo* and the reaction mass was dissolved in tetrahydrofuran (20 mL) and triethylamine (0.8 mL, 5.85 mmol) added, then stirred for 24 hours at room temperature. The material was purified by flash chromatography on silica gel (petroleum ether: ethyl acetate, 7:3) to afford a pale white powder (0.761 g, 4.0 mmol) in 89 % yield.

¹H NMR (500MHz, CDCl₃): δ = 6.90 (s, 1H, C=CH), 3.09 (s, 3H, N-CH₃); ¹³C NMR (125MHz, CDCl₃): δ = 168.6 (C=O), 165.4 (C=O), 131.9 (-C=CH-), 131.4 ((Br)C=C-), 24.7 (-N-CH₃); IR (solid, cm⁻¹): 3106 (s), 1708 (s); MS (CI) *m*/*z*, (%): 192 (⁸¹M+, 99), 190(⁷⁹M+, 100); Mass calculated for C₅H₅O₂N⁷⁹Br: 189.95037. Found: 189.95052; m.p: 77-79°C.

### Example 1: Preparation of N-Boc-Cys(Mal)-OMe (compound 4)

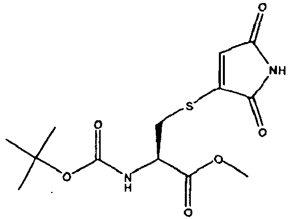

To a stirring solution of N-Boc-Cys-OMe (compound 3) (36 mg, 0.153 mmol) and sodium acetate (13 mg, 0.153 mmol) in methanol (3mL) was added bromomaleimide (30 mg, 0.169 mmol) in methanol (3 mL). After 1 minute solvent was removed in *vacuo.* The material was purified by flash chromatography on silica gel (petroleum ether: ethyl acetate, gradient elution from 9:1 to 7:3) to afford a pale yellow powder (51 mg, 0.153 mmol) in 100 % yield.

¹H NMR (500MHz, CDCl₃): δ =7.63 (s, 1H, NH), 6.27 (s, 1H, C=CH), 5.40 (d, 1H, *J* = 6.8, NH), 4.67 (ddd, 1H, J= 6.8, 5.4 and 5.1, -HN-C*H*-C(O)-), 3.80 (s, 3H, O-CH₃), 3.48 (dd, 1H, *J* = 13.8 and 5.1, -S-CHH-), 3.62 (dd, 1H, *J* = 14.1 and 5.4, -S-CH*H*-) 1.45 (s, 9H, 3 x CH₃); ¹³C NMR (125MHz, CDCl₃): δ = 170.2 (C=O), 168.9 (C=O), 167.6 (C=O), 155.2 (C=O), 155.9 (-C=CH-), 119.7 (-C=CH-), 81.1 ((CH₃)*C*O-), 53.3 (O-CH₃), 52.7 (CH), 34.0 (CH₂), 28.3 (3 x CH₃); IR (solid, cm⁻¹) 3236 (w), 1715 (s); MS (CI+) *mlz,* (%): 331 (M+H, 5), 275 (20), 231 (100); Mass calculated for [C₁₃H₁₈O₆N₂S]+H: 331.09638. Found: 331.09684; ²⁰α_{D}: -41.9° (c = 1.0, Methanol); m.p. 145-147°C.

### Example 2: Preparation of N-Boc-Cys(N-Me-Mal)-OMe (compound 5)

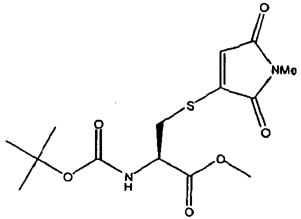

To a stirring solution of N-Boc-Cys-OMe (32 mg, 0.136 mmol) in methanol (4 mL) was added sodium acetate (82 mg, 0.408 mmol). To this was added N-methyl bromomaleimide (25.8 mg, 0.136 mmol) in methanol (4 mL) over 10 minutes. The reaction turned light yellow. The solvent was removed *in vacuo* and the residue was purified by flash chromatography on silica gel (petroleum ether: ethyl acetate, gradient elution from 9:1 to 7:3) to afford a pale white powder (39.3 mg, 0.114 mmol) in 84 % yield.

¹H NMR (500MHz, CDCl₃): δ = 6.26 (s, 1H, C=CH), 5.36 (d, 1H, J = 6.3, NH), 4.66 (m, 1H, -HN-C*H*-), 3.79 (s, 3H, O-CH₃), 3.46 (dd, 1H, J= 5.2 and 5.0, -S-CHH-), 3.35 (dd, 1H, *J* = 13.7 and 5.1, -S-CH*H*-), 3.00 (s, 3H, -N-CH₃), 1.44 (s, 9H, 3 x CH₃); ¹³C NMR (125MHz, CDCl₃): δ = 170.2 (C=O), 169.5 (C=O), 167.9 (C=O), 155.0 (C=O), 149.9(-*C*=CH-), 118.7 (-C=CH-), 80.9 ((CH₃)₃*C*O-), 53.1 (O-CH₃), 52.7 (CH), 33.8 (CH₂), 28.3 (3 x CH₃), 24.1 (-N-CH₃); IR (solid, cm⁻¹) 3367.8, 2977.1, 1694.7; MS (ES+) *m*/*z*, (%): 367(46), 311 (M, 100); Mass calculated for C₁₄H₂₀N₂O₆NaS: 367.0940. Found: 367.0931; ²⁰α_{D}: -18.55° (c = 1.0, Methanol); m.p. 101-103°C.

### Example 3: Demonstration of selectivity of the bromomaleimide reagent for N-Boc-Cys-OMe compared to propylamine

To a stirring solution of N-Boc-Cys-OMe (36 mg, 0.153 mmol) and propylamine (10 µL, 0.153 mmol) in methanol (3m L) was added bromomaleimide (30 mg, 0.169 mmol) in methanol (3 mL). After 1 minute solvent was removed *in vacuo.* The material was purified by flash chromatography on silica gel (petroleum ether: ethyl acetate, gradient elution from 9:1 to 7:3) to afford a pale yellow powder (51 mg, 0.153 mmol) in 100%. Data matched those obtained above for N-Boc-Cys(Mal)-OMe 4.

### Example 4: Cleavage of N-Boc-Cys(Mal)-OMe to regenerate N-Boc-Cys-OMe

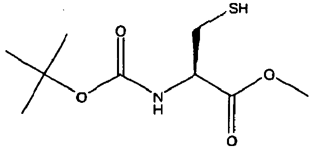

To a stirring solution of 4 (50 mg, 0.151 mmol) in dimethylformamide (2 mL) was added 20 mL of an aqueous buffer (150 mM NaCl, 100 mM NaH₂PO₄, pH 8.0). Tris(2-carboxyethyl)phosphine (430 mg 1.51 mmol) in 20 mL of an aqueous buffer (150 mM NaCl, 100 mM NaH₂PO₄, pH 8.0) was added to the solution. After 5 minutes the aqueous solution was extracted with ethyl acetate (3 x 25 mL), washed with saturated lithium chloride solution (5 x 25mL), water (25 mL) and brine (25 mL) and dried over MgSO₄. Solvent was removed *in vacuo* to afford a colourless oil (34.5 mg, 0.148 mmol) in 98% yield. ¹H and ¹³C NMR of this oil showed it to be the commercially available N-Boc-cysteine methyl ester 3.

### Example 5: Reaction of 2,3-dibromomaleimide with somatostatin

Somatostatin is peptide hormone which is known to exist in a form in which two cysteine residues within the molecule are attached via a disulfide bridge.

1 mg of lyophilised somatostatin (Sigma-Aldrich) was resolubilised in 2 ml of 50 mM NaHPO₄⁻, pH 6.2, 40 % MeCN, 2.5 % DMF. 500 µl were transferred to a Eppendorf reaction tube and diluted in the same buffer to a final concentration of 0.25 mg/ml (152.6 µM). 2.0 equivalents of TCEP (100x stock solution in 50 mM NaHPO₄⁻, pH 6.2, 40% MeCN) were added and the reaction incubated for 1 hour at ambient temperature. After reduction of the disulfide bond 1.4 equivalents of 2,3-dibromomaleimide (Sigma-Aldrich, 100x stock solution in 50 mM NaHPO₄⁻, pH 6.2, 40 % MeCN, 2.5 % DMF) were added, the solution gently mixed and incubated for a further 12 h at 4°C.

Maleimide-bridged somatostatin was detected by LC-ESI-MS (ES⁺/ES⁻). Controls included untreated peptide and somatostatin treated with 2,3-dibromomaleimide or TCEP only. Complete reduction was detected by the reaction of TCEP-treated peptide with maleimide (Sigma-Aldrich, 100x stock solution in 50 mM NaHPO₄⁻, pH 6.2, 40 % MeCN, 2.5 % DMF).

### Experimental data:

Untreated somatostatin: [ES+] 1638.04 (m/z 1), 819.82 (m/z 2), 546.95 (m/z 3). Maleimide-bridged somatostatin: [ES+] 1734.14 Da (m/z 1), 867.40 Da (m/z 2), 578.73 (m/z 3).

### Reference Example 3: Expression of GrB2-SH2 Domain L111C

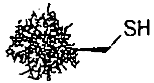

The protein GrB2-SH2 domain L111C was used as a model protein. This model protein contains a single cysteine residue.

LC-MS was performed on a Waters Acquity uPLC connected to Waters Acquity Single Quad Detector (SQD). Column: Acquity uPLC BEH C18 1.7µm 2.1 x 50 mm. Wavelength: 254 nm. Mobile Phase: 95:5 Water (0.1% Formic Acid): MeCN (0.1% Formic Acid) Gradient over 4 min (to 5:95 Water (0.1 % Formic Acid): MeCN (0.1 % Formic Acid). Flow Rate: 0.6 mL/min. MS Mode: ES+. Scan Range: m/z = 85-2000. Scan time: 0.25 sec. Data obtained in continuum mode. The electrospray source of the MS was operated with a capillary voltage of 3.5 kV and a cone voltage of 50 V. Nitrogen was used as the nebulizer and desolvation gas at a total flow of 600 L/h. Total mass spectra were reconstructed from the ion series using the MaxEnt 1 algorithm preinstalled on MassLynx software.

The model protein was over-expressed in *E. coli,* and the hexa-His-tagged protein purified using both Ni-affinity chromatography and size-exclusion chromatography *via* standard techniques. Analysis using LC-MS showed a single protein species of mass 14169 which corresponds to the desired protein.

To a solution of model protein (100 µL, [Protein] 2.0 mg/mL, 100 mM sodium phosphate, 150 mM NaCl, pH 8.0) at 0°C was added Ellman's reagent (5 µL, 282 mM solution in H₂O) at 0°C. The mixture was vortexed for 1 s and maintained at 0 °C for 10 mins, after which the mixture was analysed by LC-MS. Analysis showed that a single reaction had occurred yielding a single product with a mass of 14366 showing that C111 was available for functionalisation.

### Example 6: Reaction of GrB2-SH2 Domain L111C with bromomaleimide

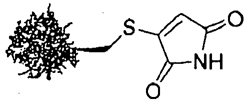

To a solution of model protein (100 µL, [Protein] 2.0 mg/mL, 100 mM sodium phosphate, 150 mM NaCl, pH 8.0) at 0 °C was added bromomaleimide (5 µL, 2.82 mM solution in DMF). The mixture was vortexed for 1 s then maintained at 0 °C for 1 h. Analysis using LC-MS showed a single protein species of mass 14265 which corresponds to the desired protein.

The mixture was treated with Ellman's reagent (5 µL, 282 mM solution in H₂O) at 0 °C. The mixture was vortexed for 1 s and maintained at 0 °C for 10 mins after which the mixture was analysed by LC-MS. Analysis showed that no reaction with Ellman's reagent was evident, highlighting that bromomaleimide functionalisation had occurred at C111.

### Example 7: Reaction of GrB2-SH2 Domain L111C with N-methylbromomaleimide

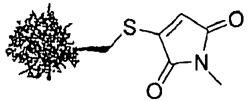

To a solution of model protein (100 µL, [Protein] 2.0 mg/mL, 100 mM sodium phosphate, 150 mM NaCl, pH 8.0) at 0 °C was added N-methylbromomaleimide (5 µL, 2.82 mM solution in DMF). The mixture was vortexed for 1 s then maintained at 0 °C for 1 h. Analysis using LC-MS showed a single protein species of mass 14278 which corresponds to the desired protein.

The mixture was treated with Ellman's reagent (5 µL, 282 mM solution in H₂O) at 0 C. The mixture was vortexed for 1 s and maintained at 00 C for 10 mins after which the mixture was analysed by LC-MS. Analysis showed that no reaction with Ellman's reagent was evident, highlighting that N-methylbromomaleimide functionalisation had occurred at C111.

### Example 8: Phosphine-Mediated Reductive Cleavage of GrB2-SH2 Domain L111C / Bromomaleimide Adduct

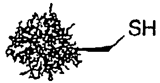

To a solution of model protein (100 µL, [Protein] 2.0 mg/mL, 100 mM sodium phosphate, 150 mM NaCl, pH 8.0) at 0 °C was added bromomaleimide (5 µL, 2.82 mM solution in DMF). The mixture was vortexed for 1 s then maintained at 0 °C for 1 h. Analysis using LC-MS showed a single protein species of mass 14265 which corresponds to protein / bromomaleimide adduct.

The mixture was treated with TCEP.HCl (5 µL, 282 mM solution in H₂O) at 0 °C. The mixture was vortexed for 1 s and maintained at 0 °C for 3 h after which the mixture was analysed by LC-MS. Analysis showed that the protein / bromomaleimide adduct had been cleanly cleaved yielding GrB2-SH2 domain L111C (mass = 14168) in 85% yield. The remaining material was unreacted protein / bromomaleimide adduct.

### Example 9: Phosphine-Mediated Reductive Cleavage of GrB2-SH2 Domain L111C / N-methylbromomaleimide Adduct

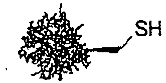

To a solution of model protein (100 µL, [Protein] 2.0 mg/mL, 100 mM sodium phosphate, 150 mM NaCl, pH 8.0) at 0 °C was added N-methylbromomaleimide (5 µL, 2.82 mM solution in DMF). The mixture was vortexed for 1 s then maintained at 0 °C for 1h. Analysis using LC-MS showed a single protein species of mass 14278 which corresponds to protein / N-methylbromomaleimide adduct.

The mixture was treated with TCEP.HCl (5 µL, 282 mM solution in H₂O) at 0 °C. The mixture was vortexed for 1 s and maintained at 0 °C for 3 h after which the mixture was analysed by LC-MS. Analysis showed that the protein / N-methylbromomaleimide adduct had been cleanly cleaved yielding GrB2-SH2 domain L111C (mass = 14168) in 85% yield. The remaining material was unreacted protein / N-methylbromomaleimide adduct.

### Example 10: Synthesis of GrB2-SH2 Domain L111C / Dibromomaleimide Adduct

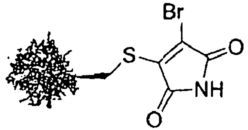

To a solution of model protein (100 µL, [Protein] 2.0 mg/mL, 100 mM sodium phosphate, 150 mM NaCl, pH 8.0) at 0 °C was added dibromomaleimide (5 µL, 2.82 mM solution in DMF). The mixture was vortexed for 1 s then maintained at 0 °C for 4 h. Analysis using LC-MS showed a single protein species of mass 14346 which corresponds to protein / dibromomaleimide adduct.

### Example 11: Synthesis of GrB2-SH2 Domain L111C / Dibromomaleimide / Glutathione Adduct

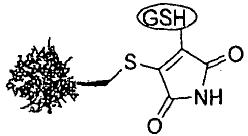

To a solution of model protein (100 µL, [Protein] 2.0 mg/mL, 100 mM sodium phosphate, 150 mM NaCl, pH 8.0) at 0°C was added dibromomaleimide (5 µL, 2.82 mM solution in DMF). The mixture was vortexed for 1 s then maintained at 0°C for 4 h. Analysis using LC-MS showed a single protein species of mass 14346 which corresponds to protein / dibromomaleimide adduct.

The mixture was treated with glutathione (5 µL, 2.82 mM solution in H₂O) at 0°C. The mixture was vortexed for 1 s and maintained at 0°C for 2 h after which the mixture was analysed by LC-MS. Analysis showed that the protein / dibromomaleimide / glutathione adduct was the only protein species present (mass = 14572).

### Example 12: Glutathione-mediated Cleavage of GrB2-SH2 Domain L111C / Dibromomaleimide / Glutathione Adduct At Physiological Relevant Glutathione Concentration (5 mM)

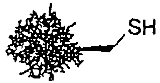

To a solution of model protein (100 µL, [Protein] 2.0 mg/mL, 100 mM sodium phosphate, 150 mM NaCl, pH 8.0) at 0 °C was added dibromomaleimide (5 µL, 2.82 mM solution in DMF). The mixture was vortexed for 1 s then maintained at 0 °C for 4 h. Analysis using LC-MS showed a single protein species of mass 14346 which corresponds to protein / dibromomaleimide adduct.

The mixture was treated with glutathione (5 µL, 2.82 mM solution in H₂O) at 0 °C. The mixture was vortexed for 1 s and maintained at 0°C for 2 h after which the mixture was analysed by LC-MS. Analysis showed that the protein / dibromomaleimide / glutathione adduct was the only protein species present (mass = 14572).

The mixture was treated with glutathione (5 µL, 100 mM solution in H₂O) at 0 °C. The mixture was vortexed for 1 s and maintained at 0°C for 4 h after which the mixture was analysed by LC-MS. Analysis showed that GrB2-SH2 domain L111C was the only protein species present (mass = 14173).

### Example 13: Preparation of N-Ac-Cys(Mal)-Benzylamine

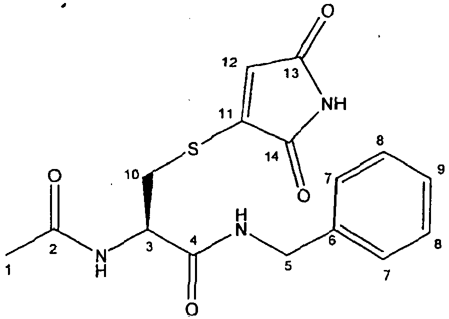

To *N-*Ac-Cys-Benzylamine (1.00 g, 4.00 mmol)above) in methanol (42 mL), was added bromomaleimide (777 mg, 4.37 mmol) in methanol (42 mL) dropwise over 5 minutes. After 10 minutes, solvent removed *in vacuo* and residue subjected to flash chromatography using 10 % ethyl acetate in petroleum ether afford the desired compound as an off-white solid (429 mg, 1.2 mmol) in 100% yield, based on 69 % recovery of the bromomaleimide. δ_{H}(500 MHz, MeOD) 7.32-7.20 (m, 5H, 5 x Ar-H), 6.45 (s, 1H, H-12), 4.71 (t, 1H, *J=* 7.3, H-3), 4.38 (d, 2H, *J=* 2.7, H₂-5), 3.40 (dd, 1H, *J =* 7.0 and 13.6, H*H*-*10),* 3.25 (dd, 1H, *J =* 7.2 and 13.6, *H*H-10), 1.99 (s, 3 H, H₃-1); δ_{C}(125 MHz, MeOD) 173.51 (C=O), 172.22 (C=O), 171.44 (C=O), 170.51 (C=O), 151.58 (C11), 139.48 (C6), 129.54 (2 x Ar-H), 128.51 (2 x Ar-H), 128.26 (C9), 121.01 (C12) 53.04 (C3), 44.25 (C5), 33.72 (C10), 22.42 (C1); IR (film, cm⁻¹) 3187 (w), 1717 (s), 1646 (s); MS (ES+) m/z (relative intensity): 370 ([M+Na], 20), 337 (50), 325 (90), 309(100); Exact Mass Calcd for [C₁₆H₁₇N₃O₄SN]+Na requires m/z 370.0873 Found 370.0852 (ES+); UV (Acetonitrile) ε₂₁₃ 19400, ε₂₄₇ = 4800 and ε₃₃₇ = 2700 cm⁻¹M⁻¹d³; White solid decomposes at 180°C.

### Example 14: Preparation of (S)-methyl 2-(tert-butoxycarbonylamino)-3-(1-(2-(5-(dimethylamino)naphthalene-1-sulfonamido)ethyl)-2,5-dioxo-2,5-dihydro-1H-pyrrol-3-ylthio)propanoate

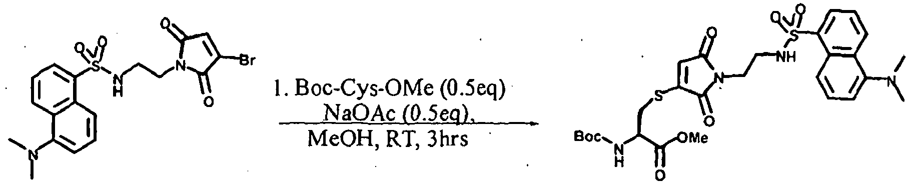

Dansyl-bromomaleimide (100mg) was dissolved in methanol (200ml) by briefly heating the stirring solution using a heat gun. To the resulting pale yellow solution, N-Boc-Cys-OMe (22µl, 0.1mmol, 0.5eq) and sodium acetate (14.5mg, 0.1mmol, 0.5eq)) were added over 3 hours. The reaction was monitored by TLC (eluent: 40%EtOAc: 60%Petroleum ether) Once the addition was complete the methanol was removed in *vacuo* to yield a yellow oil. Purification by column chromatography yielded the desired product (48.29mg, 0.08 mmol, 79.7%). ¹H NMR (600MHz CDCl3) δ_{H} 8.54(d, J=8.56Hz, 1H, CH), 8.21(d, 1H, J=8.27Hz, CH), 8.13(d, 1H, J=8.57Hz, CH), 7.55(m, 2H, 2 x CH), 7.25(d, J=7.60Hz, 1H, CH), 5.92(s, 1H, CH), 4.44(m, 1H, HN-CH-CO), 3.77(s, 3H, OMe), 3.48(m, 2H, CH₂), 3.44(m, 2H, CH₂), 3.38(s, 6H, 2 xCH₃), 3.13(t, J=5.79, 2H, S-CH₂) 2.88(s, 9H, 3 x CH₃). ¹³C NMR (600MHz CDCl3): 173.08, 170.52, 169.69, 168.96 (4x C=O), 157.73 (-C=CH), 153.16, 150.71, 136.48, 131.39, 131.25, 131.18, 130.74, 130.65, 129.31, 124.35, 120.61, 119.22, 81.12, 53.58, 52.95, 45.89, 41.53, 33.98, 28.66. IR: 3324.7 cm⁻¹, 1775 cm⁻¹. [M + H]+: 605.1756, calculated; 605.1740.

### Example 15: Preparation of Glu-Cys(Mal)-Gly

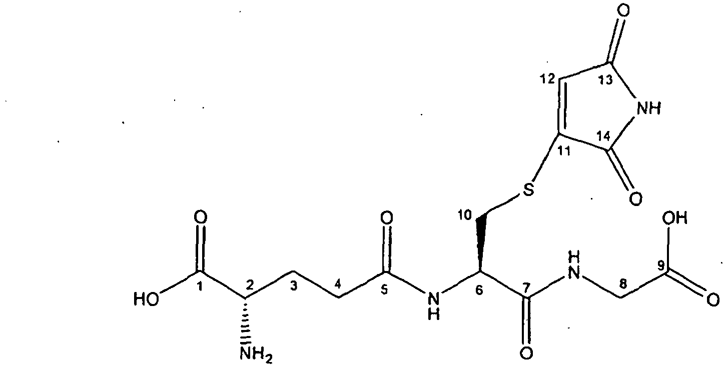

To glutathione (47 mg, 0.15 mmol) in methanol (3 mL) was added bromomaleimide (30 mg, 0.15 mmol) in methanol (3 mL). After five minutes solvent removed *in vacuo* to afford the desired compound as a thick colourless oil (62 mg, 0.15 mmol) in 100% yield. δ_{H} (500 MHz, MeOD) 6.47 (s, 1H, H-12), 4.79 (dd, 1H, *J=* 5.7 and 8.2, H-6), 4.06 (t, 1H, *J*= 6.5, H-2), 3.95 (s, 2H, H₂-8), 3.49 (dd, 1H, *J*= 5.8 and 13.9, *H*H-10), 3.29 (dd, 1H, *J =* 8.3 and 13.6, H*H-*10), 2.61 (t, 2H, *J =* 7.1, H₂-4), 2.29-2.15 (m, 2H, H₂-3); δ_{C}(125 MHz, MeOD) 174.68 (C=O), 172.81 (C=O), 172.39 (C=O), 171.89 (C=O), 171.62 (C=O), 170.59 (C=O), 151.75 (C11), 120.91 (C12), 53.79 (C6), 52.76 (C2), 42.01 (C8), 33.92 (C10) 32.42 (C4), 27.03 (C3); IR (oil, cm⁻¹) 3259 (m), 2928 (m), 1717 (s); MS (ES-) *m*/*z* (relative intensity): 401 ([M-H], 100), 272 (30); Exact Mass Calcd for [C₁₄H₁₈N₄O₈S]-H requires m/z 401.0767 Found 401.0773 (ES-); UV (Acetonitrile) ε₂₀₄ = 8100, ε₂₅₃ = 5600 and ε₃₄₂ = 1900 cm⁻¹M⁻¹d³.

### Example 16: Preparation of Preparation of Boc-Cys(MeMal)-Phe-ⁱPr

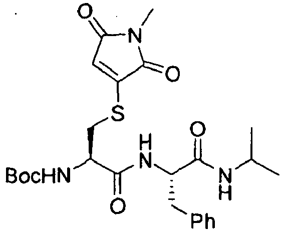

*O*-(Benzotriazol-1-yl)-*N,N,N',N'*-tetramethyluronium hexafluorophosphate (344 mg, 0.82 mmol) was added to a stirred solution of (2*R*)-2-[(*tert*-butoxycarbonyl)amino]-3-[(1-methyl-2,5-dioxo-2,5-dihydro-1*H-*pyrrol-3-yl)sulfanyl]propanoic acid (313 mg, 0.95 mmol) and 1-hydroxybenzotriazole hydrate (139 mg) in DMF (2 mL) and the reaction was stirred at 21 °C for 3 mins. A solution of (2S)-1-oxo-3-phenyl-1-(propan-2-ylamino)propan-2-ammonium trifluoroacetate (262 mg, 0.82 mmol) in DMF (1.5 mL) was added to the reaction mixture followed by *N,N-*diisopropylethylamine (294 µL, 1.64 mmol) and the reaction stirred at 21 °C for 4 h. The solvent was removed in *vacuo* and the residue dissolved in EtOAc (60 mL) and washed with I M HCl (x3), H₂O (x1), sat NaHCO₃ (x3), 10% LiCl (x1) and sat. NaCl (x1), dried (MgSO₄), filtered and the solvent *removed in vacuo.* Purification by precipitation (CHCl₃/petroleum ether 40-60) gave the desired compound as a pale brown solid (359 mg, 0.69 mmol, 84% yield): ¹H NMR (600 MHz, CD₃CN, 25 °C) δ 7.32-7.28 (m, 2H), 7.25-7.21 (m, 3H), 7.11 (d, J = 7.7 Hz, 1H), 6.46 (d, J = 6.3 Hz, 1H), 6.42 (s, 1H), 4.46 (td, J = 7.6, 6.5 Hz, 1H), 4.31 (td, J = 7.3, 6.4 Hz), 3.88 (septets of doublet, J = 6.6, 6.3 Hz, 1H), 3.30 (dd, J = 13.7, 5.8 Hz, 1H), 3.16 (dd, J = 13.7, 7.4 Hz, 1H), 2.95 (dd, J = 13.8, 7.5 Hz, 1H), 2.93 (s, 3H), 1.43 (s, 9H), 1.07 (d, J = 6.6 Hz, 3H), 1.02 (d, J = 6.6 Hz, 3H); ¹³C NMR (151 MHz, CD₃CN, 25 °C) δ 169.39, 168.84, 168.77, 167.85, 155.17, 149.33, 136.77, 129.10, 127.98, 126.30, 118.57, 79.49, 54.10, 52.62, 40.91, 37.44, 32.43, 27.15, 22.95, 21.24, 21.17; IR (thin film) 3301, 2973, 1770, 1701, 1674, 1641, 1525 cm⁻¹; LRMS (EI) 518 (24%, [M]⁺), 432 (23), 219 (33), 149 (21) 110 (27), 86 (37), 84 (100); HRMS (EI) calcd for C₂₅H₃₄N₄O₆S [M]^{+.} 518.2194, observed 518.2199.

### Example 17: Deprotection of Boc-Cys(MeMal)-Phe-ⁱPr

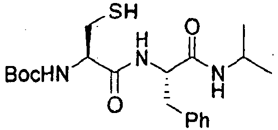

Tris(2-carboxyethyl)phosphine hydrochloride (138 mg, 0.48 mmol) in 150 mM phosphate buffer (pH 8, 25 mL) was added to a stirred solution of Boc-Cys(MeMal)-Phe-*ⁱ*Pr (50 mg, 97 µmol) in MeCN (25 mL) and the reaction stirred at 21 °C for 10 min. Synthesis of Boc-Cys-Phe-*ⁱ*Pr was confirmed by LCMS (ES⁻) 408.7 (100%).

### Reference Example 4: Cloning and Expression of Grb2-SH2 L111C Mutant

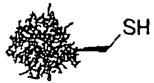

Sequence of Grb2-SH2 L111C (residues 53-163): M G I E M K P H P W F F G K I P RAKAEEMLSKQRHDGAFLIRESESAPGDFSLSVKFGND VQHFKVCRDGAGKYFLWVVKFNSLNELVDYHRSTSVS RNQQIFLRDIEQVPQQPTYVQAGSRSHHHHHHStop. Calculated mass = 14171
The DNA construct for the Grb2 SH2 domain contained the primary amino acid sequence 53-163 and was cloned on plasmid QE-60 (Qiagen). The Grb2 SH2 L111C mutant was constructed by site-directed mutagenesis (Stratagene Kit) using oligonucleotides coding for the mutated residue. Both constructs were expressed in *Escherichia coli* (M15[pREP4], Qiagen) using a T5 promoter and a C-terminal 6-His Tag was incorporated for the purification. Cultures (1 L) were grown at 37 °C in T.B. from a single colony, and expression was induced with 1.0 mM IPTG when an O.D._{λ600} of 0.9 was reached. Cultures were allowed to express protein for roughly 3 h before the cells were pelletised. Pellets were lysed in 0.1 M sodium phosphate, 300 mM NaCl, 50 mM imidazole, pH 7.2 containing a protease inhibitor cocktail (Roche). The lysate was centrifuged, and the supernatant was applied to a Ni-NTA column (Qiagen). Grb2-SH2 L111C was eluted from the Ni-NTA column with 0.1M sodium phosphate, 300 mM NaCl, 200 mM imidazole at pH 7.2. The collected Grb2 SH2 L111C was ∼95% pure as visualized by Coomassie-stained SDS-PAGE. Dimerization of Grb2 SH2 domain through domain-swapping has been previously observed. Dimeric and monomeric Grb2-SH2 were separated on a Sephacryl S-100 column (320 mL) that had been pre-equilibrated with 0.1 M sodium phosphate and 150 mM NaCl at pH 8.0. Two peaks eluted, corresponding to the molecular weights of monomer (∼14 kDa) and dimer (∼28 kDa) Grb2-SH2. Almost, 60% of the Grb2-SH2 L111C domain eluted from the column as monomer. Separated monomer and dimer were found to be surprisingly kinetically stable, as very little interconversion was seen over a course of months at 4 °C. The monomer was concentrated using Amicon® Ultra-4 centrifugal filter units (Millipore) and the final concentration of the protein was determined by absorbance at 280nm using the extinction coefficient obtained by McNemar and coworkers (15,600M⁻¹). The protein was frozen at 2 mg/mL concentration in 100 mL aliquots which were thawed as required for experiments.The mass of the monomeric protein (mass 14170) was obtained using ESI-MS.
To a solution of model protein (100 µL, [Protein] 2.0 mg/mL, 100 mM sodium phosphate, 150 mM NaCl, pH 8.0) at 0°C was added Ellman's reagent (5 µL, 282 mM solution in H₂O) at 0°C. The mixture was vortexed for 1 s and maintained at 0°C for 10 mins after which the mixture was analysed by LC-MS. Analysis showed that a single reaction had occurred yielding a single product with a mass of 14370 showing that C111 was available for functionalisation.

### Example 18: Preparation of GrB2-SH2 Domain L111C / Bromomaleimide Adduct

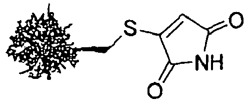

To a solution of model protein (100 µL, [Protein] 2.0 mg/mL, 100 mM sodium phosphate, 150 mM NaCl, pH 8.0) at 0°C was added bromomaleimide (5 µL, 2.82 mM solution in DMF). The mixture was vortexed for 1 s then maintained at 0°C for 1 h. Analysis using LC-MS showed that the desired product had been formed in quantitative conversion (mass 14266).

The mixture was treated with Ellman's reagent (5 µL, 282 mM solution in H₂O) at 0°C. The mixture was vortexed for 1 s and maintained at 0°C for 10 mins after which the mixture was analysed by LC-MS. Analysis showed that no reaction with Ellman's reagent was evident highlighting that bromomaleimide functionalisation had occurred at C111.

### Example 19: Preparation of GrB2-SH2 Domain L111C / N-Methylbromomaleimide Adduct

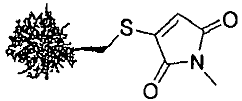

To a solution of model protein (100 µL, [Protein] 2.0 mg/mL, 100 mM sodium phosphate, 150 mM NaCl, pH 8.0) at 0°C was added N-methylbromomaleimide (5 µL, 2.82 mM solution in DMF). The mixture was vortexed for 1 s then maintained at 0°C for 1 h. Analysis using LC-MS showed that the desired product had been formed in quantitative conversion (mass 14280).

The mixture was treated with Ellman's reagent (5 µL, 282 mM solution in H₂O) at 0°C. The mixture was vortexed for 1 s and maintained at 0°C for 10 mins after which the mixture was analysed by LC-MS. Analysis showed that no reaction with Ellman's reagent was evident highlighting that N-methylbromomaleimide functionalisation had occurred at C111.

### Example 20: Phosphine-Mediated Reductive Cleavage of GrB2-SH2 Domain L111C / Bromomaleimide Adduct

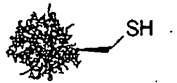

To a solution of model protein (100 µL, [Protein] 2.0 mg/mL, 100 mM sodium phosphate, 150 mM NaCl, pH 8.0) at 0°C was added bromomaleimide (5 µL, 2.82 mM solution in DMF). The mixture was vortexed for 1 s then maintained at 0°C for 1 h. Analysis using LC-MS showed a single protein species of mass 14265 which corresponded to protein / bromomaleimide adduct.

The mixture was treated with TCEP.HCl (5 µL, 282 mM solution in H₂O) at 0°C. The mixture was vortexed for 1 s and maintained at 0°C for 3 h after which the mixture was analysed by LC-MS. Analysis showed that the protein / bromomaleimide adduct had been cleanly cleaved yielding the desired product (mass = 14169) in 80% conversion

### Example 21: β-Mercaptoethanol-Mediated Reductive Cleavage of GrB2-SH2 Domain L111C / Bromomaleimide Adduct

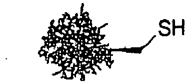

To a solution of model protein (100 µL, [Protein] 2.0 mg/mL, 100 mM sodium phosphate, 150 mM NaCl, pH 8.0) at 0°C was added bromomaleimide (5 µL, 2.82 mM solution in DMF). The mixture was vortexed for 1 s then maintained at 0°C for 1 h. Analysis using LC-MS showed a single protein species of mass 14265 which corresponded to protein / bromomaleimide adduct.

The mixture was treated with β-mercaptoethanol (5 µL, 282 mM solution in H₂O), vortexed for 1 s and maintained at 37°C for 4 h. Analysis showed that the protein / bromomaleimide adduct had been cleanly cleaved yielding the desired product (mass = 14173) in quantitative conversion.

### Example 22: Glutathione-Mediated Cleavage of GrB2-SH2 Domain L111C / Bromomaleimide Adduct

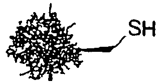

To a solution of model protein (100 µL, [Protein] 2.0 mg/mL, 100 mM sodium phosphate, 150 mM NaCl, pH 8.0) at 0°C was added bromomaleimide (5 µL, 2.82 mM solution in DMF). The mixture was vortexed for 1 s then maintained at 0°C for 1 h. Analysis using LC-MS showed a single protein species of mass 14265 which corresponded to protein / bromomaleimide adduct.

The mixture was treated with glutathione (5 µL, 282 mM solution in H₂O), vortexed for 1 s and maintained at 37°C for 4 h. Analysis showed that the protein / bromomaleimide adduct had been cleanly cleaved yielding the desired product (mass =14173) in quantitative conversion.

### Example 23: Phosphine-Mediated Reductive Cleavage of GrB2-SH2 Domain L111C / N-Methylbromomaleimide Adduct

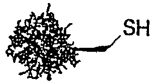

To a solution of model protein (100 µL, [Protein] 2.0 mg/mL, 100 mM sodium phosphate, 150 mM NaCl, pH 8.0) at 0°C was added N-methylbromomaleimide (5 µL, 2.82 mM solution in DMF). The mixture was vortexed for 1 s then maintained at 0°C for 1 h. Analysis using LC-MS showed a single protein species of mass 14278 which corresponded to protein / N-methylbromomaleimide adduct.

The mixture was treated with TCEP.HCl (5 µL, 282 mM solution in H₂O) at 0°C. The mixture was vortexed for 1 s and maintained at 0°C for 3 h after which the mixture was analysed by LC-MS. Analysis showed that the protein / N-methylbromomaleimide adduct had been cleanly cleaved yielding the desired product (mass = 14168) in 85% conversion.

### Example 24: β-Mercaptoethanol-Mediated Reductive Cleavage of GrB2-SH2 Domain L111C / N-Methylbromomaleimide Adduct

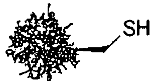

To a solution of model protein (100 µL, [Protein] 2.0 mg/mL, 100 mM sodium phosphate, 150 mM NaCl, pH 8.0) at 0°C was added N-methylbromomaleimide (5 µL, 2.82 mM solution in DMF). The mixture was vortexed for 1 s then maintained at 0°C for 1 h. Analysis using LC-MS showed a single protein species of mass 14280 which corresponded to protein / N-methylbromomaleimide adduct.

The mixture was treated with β-mercaptoethanol (5 µL, 282 mM solution in H₂O), vortexed for 1 s and maintained at 37°C for 4 h. Analysis showed that the protein / N-methylbromomaleimide adduct had been cleanly cleaved yielding the desired product (mass = 14173) in quantitative conversion.

### Example 25: Glutathione-Mediated Cleavage of GrB2-SH2 Domain L111C / N-Methylbromomaleimide Adduct

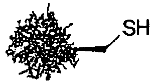

To a solution of model protein (100 µL, [Protein] 2.0 mg/mL, 100 mM sodium phosphate, 150 mM NaCl, pH 8.0) at 0°C was added N-methylbromomaleimide (5 µL, 2.82 mM solution in DMF). The mixture was vortexed for 1 s then maintained at 0°C for 1 h. Analysis using LC-MS showed a single protein species of mass 14280 which corresponded to protein / *N*-methylbromomaleimide adduct.

The mixture was treated with glutathione (5 µL, 282 mM solution in H₂O), vortexed for 1 s and maintained at 37°C for 4 h. Analysis showed that the protein / N-methylbromomaleimide adduct had been cleanly cleaved yielding the desired product (mass = 14173) in quantitative conversion.

### Example 26: Ethanedithiol-Mediated Cleavage of GrB2-SH2 Domain L111C / bromomaleimide Adduct

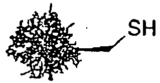

To a solution of model protein (100 µL, [Protein] 2.0 mg/mL, 100 mM sodium phosphate, 150 mM NaCl, pH 8.0) at 0°C was added bromomaleimide (5 µL, 2.82 mM solution in DMF). The mixture was vortexed for 1 s then maintained at 0°C for 1 h. Analysis using LC-MS showed a single protein species of mass 14265 which corresponded to protein / bromomaleimide adduct.

The mixture was treated with ethanedithiol (5 µL, 282 mM solution in H₂O), vortexed for 1 s and maintained at 37°C for 4 h. Analysis showed that the protein / bromomaleimide adduct had been cleanly cleaved yielding the desired product (mass = 14173) in quantitative conversion.

### Example 27: Preparation of GrB2-SH2 Domain L111C / Bromomaleimide / 2-Mercaptoethanol Adduct

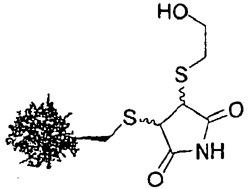

To a solution of model protein (100 µL, [Protein] 2.0 mg/mL, 100 mM sodium phosphate, 150 mM NaCl, pH 8.0) at 0°C was added bromomaleimide (5 µL, 2.82 mM solution in DMF). The mixture was vortexed for 1 s then maintained at 0°C for 1 h. Analysis using LC-MS showed a single protein species of mass 14265 which corresponded to protein / bromomaleimide adduct.

The mixture was treated with 2-mercaptoethanol (5 µL, 2.82 mM solution in H₂O) at 0°C. The mixture was vortexed for 1 s and maintained at 0°C for 3 h after which the mixture was analysed by LC-MS. Analysis showed that the protein / bromomaleimide / 2-mercaptoethanol adduct had been formed (mass = 14345) in 54% yield. The remaining material was GrB2-SH2 domain L111C.

### Example 28: Preparation of GrB2-SH2 Domain L111C / N-Methylbromomaleimide / 2-Mercaptoethanol Adduct

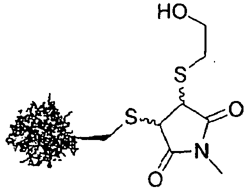

To a solution of model protein (100 µL, [Protein] 2.0 mg/mL, 100 mM sodium phosphate, 150 mM NaCl, pH 8.0) at 0°C was added N-methylbromomaleimide (5 µL, 2.82 mM solution in DMF). The mixture was vortexed for 1 s then maintained at 0°C for 1 h. Analysis using LC-MS showed a single protein species of mass 14278 which corresponded to protein / N-methylbromomaleimide adduct.

The mixture was treated with 2-mercaptoethanol (5 µL, 2.82 mM solution in H₂O) at 0°C. The mixture was vortexed for 1 s and maintained at 0°C for 3 h after which the mixture was analysed by LC-MS. Analysis showed that the desired product (mass = 14359) had been formed in 90% yield. The remaining material was GrB2-SH2 domain L111C.

### Example 29: Preparation of GrB2-SH2 Domain L111C / Bromomaleimide / Glutathione Adduct

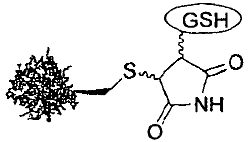

To a solution of model protein (100 µL, [Protein] 2.0 mg/mL, 100 mM sodium phosphate, 150 mM NaCl, pH 8.0) at 0°C was added bromomaleimide (5 µL, 2.82 mM solution in DMF). The mixture was vortexed for 1 s then maintained at 0°C for 1 h. Analysis using LC-MS showed a single protein species of mass 14265 which corresponds to protein / bromomaleimide adduct.

The mixture was treated with glutathione (5 µL, 2.82 mM solution in H₂O) at 0°C. The mixture was vortexed for 1 s and maintained at 0°C for 3 h after which the mixture was analysed by LC-MS. Analysis showed that the protein / bromomaleimide / glutathione adduct had been formed (mass = 14574) in 44% conversion. The remaining material was GrB2-SH2 domain L111C.

### Example 30: Preparation of GrB2-SH2 Domain L111C / N-Methylbromomaleimide / Glutathione Adduct

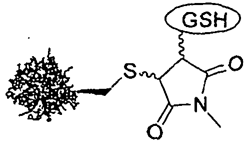

To a solution of model protein (100 µL, [Protein] 2.0 mg/mL, 100 mM sodium phosphate, 150 mM NaCl, pH 8.0) at 0°C was added N-methylbromomaleimide (5 µL, 2.82 mM solution in DMF). The mixture was vortexed for 1 s then maintained at 0°C for 1 h. Analysis using LC-MS showed a single protein species of mass 14278 which corresponded to protein / N-methylbromomaleimide adduct.

The mixture was treated with 2-mercaptoethanol (5 µL, 2.82 mM solution in H₂O) at 0°C. The mixture was vortexed for 1 s and maintained at 0°C for 3 h after which the mixture was analysed by LC-MS. Analysis showed that the protein / N-methylbromomaleimide / glutathione adduct had been formed (mass = 14588) in 95% conversion. The remaining material was GrB2-SH2 domain L111C.

### Example 31: Preparation of GrB2-SH2 Domain L111C / Dibromomaleimide Adduct

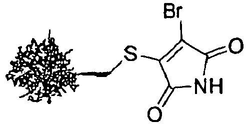

To a solution of model protein (100 µL, [Protein] 2.0 mg/mL, 100 mM sodium phosphate, 150 mM NaCl, pH 8.0) at 0°C was added dibromomaleimide (5 µL, 2.82 mM solution in DMF). The mixture was vortexed for 1 s then maintained at 0°C for 4 h. Analysis using LC-MS showed that the desired product had been formed in quantitative yield (mass 14345).

### Example 32: 2-Mercaptoethanol-mediated Reductive Cleavage of the GrB2-SH2 Domain L111C / Dibromomaleimide Adduct

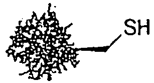

To a solution of model protein (100 µL, [Protein] 2.0 mg/mL, 100 mM sodium phosphate, 150 mM NaCl, pH 8.0) at 0°C was added dibromomaleimide (5 µL, 2.82 mM solution in DMF). The mixture was vortexed for 1 s then maintained at 0°C for 4 h. Analysis using LC-MS showed a single protein species of mass 14346 which corresponded to protein / dibromomaleimide adduct.

The mixture was treated with 2-mercaptoethanol (5 µL, 282 mM solution in H₂O) at 0°C. The mixture was vortexed for 1 s and maintained at 0°C for 4 h after which the mixture was analysed by LC-MS. Analysis showed that the protein / bromomaleimide adduct had been cleanly cleaved yielding the desired product (mass = 1417) in quantitative yield yield.

### Example 33: Glutathione-mediated Reductive Cleavage of the GrB2-SH2 Domain L111C / Dibromomaleimide Adduct

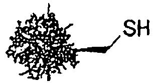

To a solution of model protein (100 µL, [Protein] 2.0 mg/mL, 100 mM sodium phosphate, 150 mM NaCl, pH 8.0) at 0°C was added dibromomaleimide (5 µL, 2.82 mM solution in DMF). The mixture was vortexed for 1 s then maintained at 0°C for 4 h. Analysis using LC-MS showed a single protein species of mass 14346 which corresponded to protein / dibromomaleimide adduct.

The mixture was treated with glutathione (5 µL, 282 mM solution in H₂O) at 0°C. The mixture was vortexed for 1 s and maintained at 0°C for 4 h after which the mixture was analysed by LC-MS. Analysis showed that the protein / bromomaleimide adduct had been cleanly cleaved yielding the desired product (mass = 14170) in quantitative yield.

### Example 34: Preparation of GrB2-SH2 Domain L111C / Dibromomaleimide / Glutathione Adduct

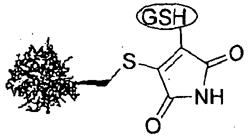

To a solution of model protein (100 µL, [Protein] 2.0 mg/mL, 100 mM sodium phosphate, 150 mM NaCl, pH 8.0) at 0°C was added dibromomaleimide (5 µL, 2.82 mM solution in DMF). The mixture was vortexed for 1 s then maintained at 0°C for 4 h. Analysis using LC-MS showed a single protein species of mass 14346 which corresponded to protein / dibromomaleimide adduct.

The mixture was treated with glutathione (5 µL, 2.82 mM solution in H₂O) at 0°C. The mixture was vortexed for 1 s and maintained at 0°C for 2 h after which the mixture was analysed by LC-MS. Analysis showed that the desired product had been formed (mass = 14573) in quantitative conversion.

### Example 35: Preparation of GrB2-SH2 Domain L111C / Dibromomaleimide / β-1-Thioglucose Adduct

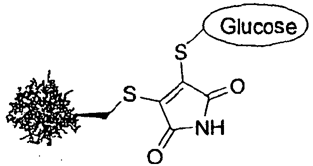

To a solution of model protein (100 µL, [Protein] 2.0 mg/mL, 100 mM sodium phosphate, 150 mM NaCl, pH 8.0) at 0°C was added dibromomaleimide (5 µL, 2.82 mM solution in DMF). The mixture was vortexed for 1 s then maintained at 0°C for 4 h. Analysis using LC-MS showed a single protein species of mass 14346 which corresponded to protein / dibromomaleimide adduct.

The mixture was treated with β-1-thioglucose, sodium salt (5 µL, 2.82 mM solution in H₂O) at 0°C. The mixture was vortexed for 1 s and maintained at 0°C for 2 h after which the mixture was analysed by LC-MS. Analysis showed that the desired product (mass = 14461) was formed in near quantitative yield.

### Example 36: Glutathione-mediated Cleavage of GrB2-SH2 Domain L-111C / Dibromomaleimide / Glutathione Adduct

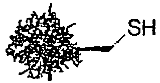

To a solution of model protein (100 µL, [Protein] 2.0 mg/mL, 100 mM sodium phosphate, 150 mM NaCl, pH 8.0) at 0°C was added dibromomaleimide (5 µL, 2.82 mM solution in DMF). The mixture was vortexed for 1 s then maintained at 0°C for 4 h. Analysis using LC-MS showed a single protein species of mass 14346 which corresponded to protein / dibromomaleimide adduct.

The mixture was treated with glutathione (5 µL, 2.82 mM solution in H₂O) at 0°C. The mixture was vortexed for 1 s and maintained at 0°C for 2 h after which the mixture was analysed by LC-MS. Analysis showed that the protein / dibromomaleimide / glutathione adduct was the only protein species present (mass = 14573).

The mixture was treated with glutathione (5 µL, 282 mM solution in H₂O) at 0°C. The mixture was vortexed for 1 s and maintained at 0°C for 4 h after which the mixture was analysed by LC-MS. Analysis showed that the desired product (mass = 14173) was formed in quantitative yield.

### Example 37: Glutathione-mediated Cleavage of GrB2-SH2 Domain L111C / Dibromomaleimide / Glutathione Adduct At Physiologically Relevant Glutathione Concentration (5 mM)

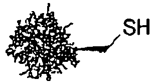

To a solution of model protein (100 µL, [Protein] 2.0 mg/mL, 100 mM sodium phosphate, 150 mM NaCl, pH 8.0) at 0°C was added dibromomaleimide (5 µL, 2.82 mM solution in DMF). The mixture was vortexed for 1 s then maintained at 0°C for 4 h. Analysis using LC-MS showed a single protein species of mass 14346 which corresponded to protein / dibromomaleimide adduct.

The mixture was treated with glutathione (5 µL, 2.82 mM solution in H₂O) at 0°C. The mixture was vortexed for 1 s and maintained at 0°C for 2 h after which the mixture was analysed by LC-MS. Analysis showed that the protein / dibromomaleimide / glutathione adduct was the only protein species present (mass = 14573).

The mixture was treated with glutathione (5 µL, 100 mM solution in H₂O) at 0°C. The mixture was vortexed for 1 s and maintained at 0°C for 4 h after which the mixture was analysed by LC-MS. Analysis showed that the desired product (mass = 14173) was formed in quantitative yield.

### Example 38: Glutathione-mediated Cleavage of GrB2-SH2 Domain L111C / Dibromomaleimide / Glutathione Adduct At Physiologically Relevant Glutathione Concentration (1 mM)

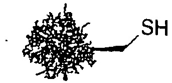

To a solution of model protein (100 µL, [Protein] 2.0 mg/mL, 100 mM sodium phosphate, 150 mM NaCl, pH 8.0) at 0°C was added dibromomaleimide (5 µL, 2.82 mM solution in DMF). The mixture was vortexed for 1 s then maintained at 0°C for 4 h. Analysis using LC-MS showed a single protein species of mass 14346 which corresponded to protein / dibromomaleimide adduct.

The mixture was treated with glutathione (5 µL, 2.82 mM solution in H₂O) at 0°C. The mixture was vortexed for 1 s and maintained at 0°C for 2 h after which the mixture was analysed by LC-MS. Analysis showed that the protein / dibromomaleimide / glutathione adduct was the only protein species present (mass = 14573).

The solution of protein / dibromomaleimide / glutathione adduct was subjected to a buffer swap (Micro Bio-Spin 6 Chromatography Column, Bio-Rad) yielding the adduct (95 *µ*L, [adduct] 0.2 mg/mL, 20 mM HEPES, 100 mM KCl, 1 mM MgCl2, 1 mM EDTA, pH 7.4). To this was added glutathione (5 *µ*L, 20 mM solution in 20 mM HEPES, 100 mM KCl, 1 mM MgCl2, 1 mM EDTA, pH 7.4). The mixture was vortexed for 1 s then maintained at 37 °C for 4 h. Analysis showed that Grb2-SH2 (L111C) was formed (mass) 14170) in quantitative conversion.

### Example 3 9: β-Mercaptoethanol-mediated Cleavage of GrB2-SH2 Domain L111C / Dibromomaleimide / Glutathione Adduct

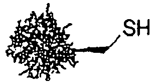

To a solution of model protein (100 µL, [Protein] 2.0 mg/mL, 100 mM sodium phosphate, 150 mM NaCl, pH 8.0) at 0°C was added dibromomaleimide (5 µL, 2.82 mM solution in DMF). The mixture was vortexed for 1 s then maintained at 0°C for 4 h. Analysis using LC-MS showed a single protein species of mass 14346 which corresponded to protein / dibromomaleimide adduct.

The mixture was treated with glutathione (5 µL, 2.82 mM solution in H₂O) at 0°C. The mixture was vortexed for 1 s and maintained at 0°C for 2 h after which the mixture was analysed by LC-MS. Analysis showed that the protein / dibromomaleimide / glutathione adduct was the only protein species present (mass = 14573).

The mixture was treated with β-mercaptoethanol (5 µL, 282 mM solution in H₂O) at 0°C. The mixture was vortexed for 1 s and maintained at 0°C for 4 h after which the mixture was analysed by LC-MS. Analysis showed that the desired product (mass = 14172) was formed in quantitative conversion.

### Example 40: Glutathione-mediated Cleavage of GrB2-SH2 Domain L111C / Dibromomaleimide / β-1-thioglucose Adduct

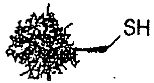

To a solution of model protein (100 µL, [Protein] 2.0 mg/mL, 100 mM sodium phosphate, 150 mM NaCl, pH 8.0) at 0°C was added dibromomaleimide (5 µL, 2.82 mM solution in DMF). The mixture was vortexed for 1 s then maintained at 0°C for 4 h. Analysis using LC-MS showed a single protein species of mass 14346 which corresponded to protein / dibromomaleimide adduct.

The mixture was treated with β-1-thioglucose (5 µL, 2.82 mM solution in H₂O) at 0°C. The mixture was vortexed for 1 s and maintained at 0°C for 2 h after which the mixture was analysed by LC-MS. Analysis showed that the protein / dibromomaleimide / β-1-thioglucose adduct was the only protein species present ( mass = 14461).

The mixture was treated with glutathione (5 µL, 282 mM solution in H₂O) at 0°C. The mixture was vortexed for 1 s and maintained at 0°C for 4 h after which the mixture was analysed by LC-MS. Analysis showed that desired product was formed (mass = 14173) in quantitative conversion.

### Example 41: β-Mercaptoethanol-mediated Cleavage of GrB2-SH2 Domain L111C / Dibromomaleimide / β-1-thioglucose Adduct

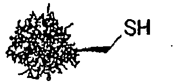

To a solution of model protein (100 µL, [Protein] 2.0 mg/mL, 100 mM sodium phosphate, 150 mM NaCl, pH 8.0) at 0°C was added dibromomaleimide (5 µL, 2.82 mM solution in DMF). The mixture was vortexed for 1 s then maintained at 0°C for 4 h. Analysis using LC-MS showed a single protein species of mass 14346 which corresponded to protein / dibromomaleimide adduct.

The mixture was treated with β-1-thioglucose (5 µL, 2.82 mM solution in H₂O) at 0°C. The mixture was vortexed for 1 s and maintained at 0°C for 2 h after which the mixture was analysed by LC-MS. Analysis showed that the protein / dibromomaleimide / β-1-thioglucose adduct was the only protein species present ( mass = 1446).

The mixture was treated with β-mercaptoethanol (5 µL, 282 mM solution in H₂O) at 0°C. The mixture was vortexed for 1 s and maintained at 0°C for 4 h after which the mixture was analysed by LC-MS. Analysis showed that desired product was formed ( mass = 14172) in quantitative conversion.

### Example 42: Preparation of GrB2-SH2 Domain L111C / N-Phenylbromomaleimide Adduct

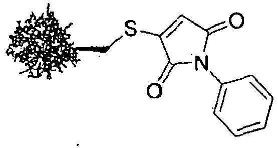

To a solution of model protein (100 µL, [Protein] 2.0 mg/mL, 100 mM sodium phosphate, 150 mM NaCl, pH 8.0) at 0°C was added *N*-phenylbromomaleimide (5 µL, 2.82 mM solution in DMF). The mixture was vortexed for 1 s then maintained at 0°C for 1 h. Analysis using LC-MS showed that the desired product had been formed in quantitative yield (mass 14351).

### Example 43: Preparation of GrB2-SH2 Domain L111C / N-Phenyldibromomaleimide Adduct

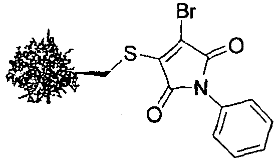

To a solution of model protein (100 µL, [Protein] 2.0 mg/mL, 20 mM MES, 150 mM NaCl, pH 6) at 0°C was added *N*-phenylbromomaleimide (5 µL, 2.82 mM solution in DMF). The mixture was vortexed for 1 s then maintained at 0°C for 1 h. Analysis using LC-MS showed that the desired product had been formed in quantitative yield (mass 14431).

### Example 44: β-Mercaptoethanol-mediated Cleavage of GrB2-SH2 Domain L111C / N-Phenyldibromomaleimide Adduct

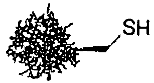

To a solution of model protein (100 µL, [Protein] 2.0 mg/mL, 20 mM MES, 150 mM NaCl, pH 6) at 0°C was added *N*-phenylbromomaleimide (5 µL, 2.82 mM solution in DMF). The mixture was vortexed for 1 s then maintained at 0°C for 1 h. Analysis using LC-MS showed that protein / N-phenyldibromomaleimide adduct had been formed in quantitative yield (mass 14431).

The mixture was treated with β-mercaptoethanol (5 µL, 282 mM solution in H₂O) at 0°C. The mixture was vortexed for 1 s and maintained at 0°C for 4 h after which the mixture was analysed by LC-MS. Analysis showed that desired product was formed ( mass = 14179) in quantitative conversion.

### Example 45: Preparation of GrB2-SH2 Domain L111C / N-Phenyldibromomaleimide / β-1-thioglucose Adduct

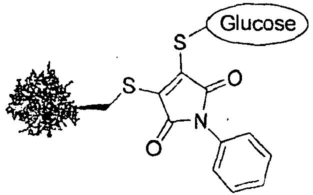

To a solution of model protein (100 µL, [Protein] 2.0 mg/mL, 20 mM MES, 150 mM NaCl, pH 6) at 0°C was added *N*-phenylbromomaleimide (5 µL, 2.82 mM solution in DMF). The mixture was vortexed for 1 s then maintained at 0°C for 1 h. Analysis using LC-MS showed that the protein / *N*-phenyldibromomaleimide adduct had been formed in quantitative yield (mass 14431).

The mixture was treated with β-1-thioglucose (5 µL, 2.82 mM solution in H₂O) at 0°C. The mixture was vortexed for 1 s and maintained at 0°C for 2 h after which the mixture was analysed by LC-MS. Analysis showed that the protein / N-phenyldibromomaleimide / β-1-thioglucose adduct was the only protein species present (mass = 14547).

### Example 46: β-Mercaptoethanol-mediated Cleavage of GrB2-SH2 Domain L111C / N-Phenyldibromomaleimide / β-1-thioglucose Adduct

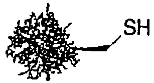

To a solution of model protein (100 µL, [Protein] 2.0 mg/mL, 20 mM MES, 150 mM NaCl, pH 6) at 0°C was added *N*-phenylbromomaleimide (5 µL, 2.82 mM solution in DMF). The mixture was vortexed for 1 s then maintained at 0°C for 1 h. Analysis using LC-MS showed that the protein / *N*-phenyldibromomaleimide adduct had been formed in quantitative yield (mass 14431).

The mixture was treated with β-1-thioglucose (5 µL, 2.82 mM solution in H₂O) at 0°C. The mixture was vortexed for 1 s and maintained at 0°C for 2 h after which the mixture was analysed by LC-MS. Analysis showed that the protein / N-phenyldibromomaleimide / β-1-thioglucose adduct was the only protein species present (mass = 14547).

The mixture was treated with β-mercaptoethanol (5 µL, 282 mM solution in H₂O) at 0°C. The mixture was vortexed for 1 s and maintained at 0°C for 4 h after which the mixture was analysed by LC-MS. Analysis showed that desired product was formed ( mass = 14178) in quantitative conversion.

### Example 47: Preparation of GrB2-SH2 Domain L111C / Biotin-PEG-bromomaleimide Adduct

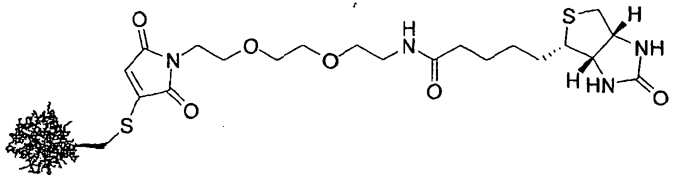

To a solution of model protein (100 µL, [Protein] 2.0 mg/mL, 100 mM sodium phosphate, 150 mM NaCl, pH 8.0) at 0°C was added biotin-PEG-bromomaleimide (5 µL, 2.82 mM solution in DMF). The mixture was vortexed for 1 s then maintained at 0°C for 1 h. Analysis using LC-MS showed that the desired product had been formed in quantitative yield (mass 14634).

### Example 48: β-Mercaptoethanol-mediated Cleavage of GrB2-SH2 Domain L111C / Biotin-PEG-bromomaleimide Adduct

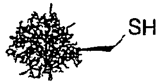

To a solution of model protein (100 µL, [Protein] 2.0 mg/mL, 100 mM sodium phosphate, 150 mM NaCl, pH 8.0) at 0°C was added biotin-PEG-bromomaleimide (5 µL, 2.82 mM solution in DMF). The mixture was vortexed for 1 s then maintained at 0°C for 1 h. Analysis using LC-MS showed that the protein / biotin-PEG-bromomaleimide adduct had been formed in quantitative yield (mass 14634).

The mixture was treated with β-mercaptoethanol (5 µL, 282 mM solution in H₂O) at 37°C. The mixture was vortexed for 1 s and maintained at 37°C for 4 h after which the mixture was analysed by LC-MS. Analysis showed that desired product was formed ( mass = 14180) in quantitative conversion.

### Example 49: Preparation of GrB2-SH2 Domain L111C / Biotin-PEG-dibromomaleimide Adduct

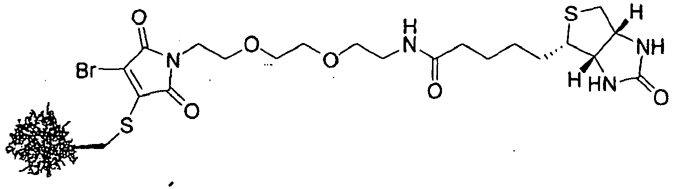

To a solution of model protein (100 µL, [Protein] 2.0 mg/mL, 100 mM sodium phosphate, 150 mM NaCl, pH 8.0) at 0°C was added biotin-PEG-dibromomaleimide (5 µL, 2.82 mM solution in DMF). The mixture was vortexed for 1 s then maintained at 0°C for 2 h. Analysis using LC-MS showed that the desired product had been formed in >80% yield (mass 14701).

### Example 50: β-Mercaptoethanol-mediated Cleavage of GrB2-SH2 Domain L111C / Biotin-PEG-dibromomaleimide Adduct

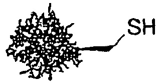

To a solution of model protein (100 µL, [Protein] 2.0 mg/mL, 100 mM sodium phosphate, 150 mM NaCl, pH 8.0) at 0°C was added biotin-PEG-dibromomaleimide (5 µL, 2.82 mM solution in DMF). The mixture was vortexed for 1 s then maintained at 0°C for 1 h. Analysis using LC-MS showed that the protein / biotin-PEG-dibromomaleimide adduct had been formed in >80% conversion (mass 14701).

The mixture was treated with β-mercaptoethanol (5 µL, 282 mM solution in H₂O) at 0°C. The mixture was vortexed for 1 s and maintained at 0°C for 4 h after which the mixture was analysed by LC-MS. Analysis showed that desired product was formed ( mass = 14171) in >80% conversion.

### Example 51: Pull-Down and Release of GrB2-SH2 Domain L111C / Biotin-PEG-bromomaleimide Adduct onto Neutravidin Coated Agarose Beads

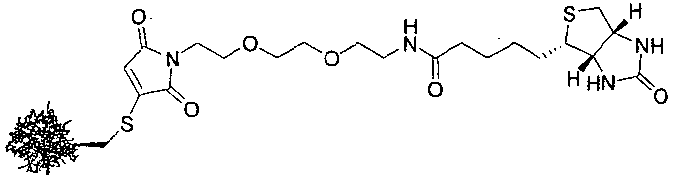

To a solution of model protein (200 µL, [Protein] 1.0 mg/mL, 100 mM sodium phosphate, 150 mM NaCl, pH 8.0) at 0°C was added biotin-PEG-bromomaleimide (5 µL, 2.82 mM solution in DMF). The mixture was vortexed for 1 s then maintained at 0°C for 1 h. Analysis using LC-MS showed that the desired product had been formed in quantitative yield (mass 14634).

Protein / biotin-PEG-bromomaleimide adduct (200 µL) and unmodified model protein (200 µL) were washed independently with PBS buffer (3 x 500 µL) in a concentrator (Vivaspin, cut off 10k) yielding protein solutions (300 µL) **(In).** For each of the protein solutions obtained, neutravidin-coated agarose beads (750 µL of 50% aqueous slurry) were washed with PBS (2 x 500 µL). Protein solution (300 µL) was then added to the beads and the mixture incubated at 4°C for 30 mins. The mixture was centrifuged and the flow through **(FT)** collected. The beads were washed with PBS (2 x 500 µL) and both wash fractions collected (**W1** and **W2).** Protein was released from the beads by incubation in PBS (300 µL) containing β-mercaptoethanol (25 mM) for 2 h at 37°C. The sample was centrifuged and the eluant (**El**) containing cleaved GrB2-SH2 domain L111C collected. The results are shown in Figure 1.

The amount of protein recovered was determined as 44% by comparison with a protein series dilution *via* densitometry. However, correcting for irreversibly physisorbed protein (determined using the unmodified protein control) the corrected recovery was 71 %.

### Example 52: Preparation of GrB2-SH2 Domain L111C / N-Fluorescein bromomaleimide Adduct

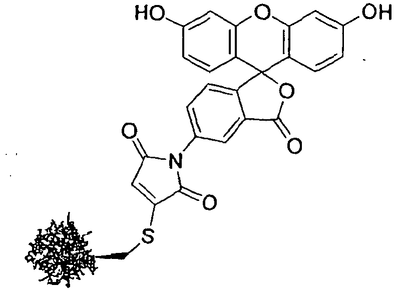

To a solution of model protein (100 µL, [Protein] 2.0 mg/mL, 100 mM sodium phosphate, 150 mM NaCl, pH 7.0) at 0°C was added *N*-fluorescein bromomaleimide (5 µL, 2.82 mM solution in DMF). The mixture was vortexed for 1 s then maintained at 0°C for 1 h. Analysis using LC-MS showed that the desired product had been formed in 90% conversion (mass 14597).

### Example 53: β-Mercaptoethanol-mediated Cleavage of GrB2-SH2 Domain L111C / N-Fluorescein bromomaleimide Adduct

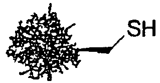

To a solution of model protein (100 µL, [Protein] 2.0 mg/mL, 100 mM sodium phosphate, 150 mM NaCl, pH 7.0) at 0°C was added *N*-fluorescein bromomaleimide (5 µL, 2.82 mM solution in DMF). The mixture was vortexed for 1 s then maintained at 0°C for 1 h. Analysis using LC-MS showed that the protein / fluorescein bromomaleimide adduct had been formed in 90% conversion (mass 14597).

The mixture was treated with β-mercaptoethanol (5 µL, 282 mM solution in H₂O) at 37°C. The mixture was vortexed for 1 s and maintained at 37°C for 4 h after which the mixture was analysed by LC-MS. Analysis showed that desired product was formed ( mass = 14171) in 87% conversion.

### Example 54: Preparation of GrB2-SH2 Domain L111C / N-Fluorescein dibromomaleimide Adduct

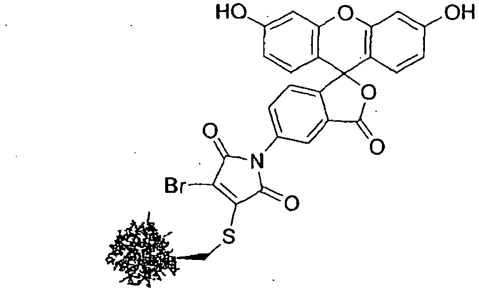

To a solution of model protein (100 µL, [Protein] 2.0 mg/mL, 100 mM sodium phosphate, 150 mM NaCl, pH 8.0) at 0°C was added *N*-fluorescein dibromomaleimide (5 µL, 2.82 mM solution in DMF). The mixture was vortexed for 1 s then maintained at 0°C for 1 h. Analysis using LC-MS showed that the desired product had been formed in 61% conversion (mass 14675).

### Example 55: β-Mercaptoethanol-mediated Cleavage of GrB2-SH2 Domain L111C / N-Fluorescein dibromomaleimide Adduct

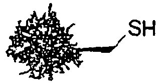

To a solution of model protein (100 µL, [Protein] 2.0 mg/mL, 100 mM sodium phosphate, 150 mM NaCl, pH 7.0) at 0°C was added N-fluorescein dibromomaleimide (5 µL, 2.82 mM solution in DMF). The mixture was vortexed for 1 s then maintained at 0°C for 1 h. Analysis using LC-MS showed that the protein / fluorescein dibromomaleimide adduct had been formed in 61 % conversion (mass 14597).

The mixture was treated with β-mercaptoethanol (5 µL, 282 mM solution in H₂O) at 37°C. The mixture was vortexed for 1 s and maintained at 37°C for 4 h after which the mixture was analysed by LC-MS. Analysis showed that desired product was formed ( mass = 14171) in 85% conversion.

### Example 56: Preparation of GrB2-SH2 Domain L111C / BrDDPD Adduct

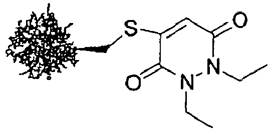

To a solution of model protein (100 µL, [Protein] 2.0 mg/mL, 100 mM sodium phosphate, 150 mM NaCl, pH 8.0) at 0°C was added BrDDPD (5 µL, 282 mM solution in DMF). The mixture was vortexed for 1 s then maintained at 37°C for 1 h. Analysis using LC-MS showed that the desired product had been formed in quantitative yield (mass 14348).

### Example 57: β-Mercaptoethanol-mediated Cleavage of GrB2-SH2 Domain L111C / BrDDPD Adduct

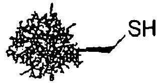

To a solution of model protein (100 µL, [Protein] 2.0 mg/mL, 100 mM sodium phosphate, 150 mM NaCl, pH 8.0) at 0°C was added BrDDPD (5 µL, 282 mM solution in DMF). The mixture was vortexed for 1 s then maintained at 37°C for 1 h. Analysis using LC-MS showed that the protein / BrDDPD adduct had been formed in quantitative yield (mass 14348).

The mixture was dialysed for 40 h at 4°C (100 mM sodium phosphate, 150 mM NaCl, pH 8.0) and treated with β-mercaptoethanol (5 µL, 2.82 M solution in H₂O) at 37°C. The mixture was vortexed for 1 s and maintained at 37°C for 2 h after which the mixture was analysed by LC-MS. Analysis showed that desired product was formed ( mass = 14180) in quantitative conversion.

### Example 58: Preparation of GrB2-SH2 Domain L111C / DiBrDDPD Adduct

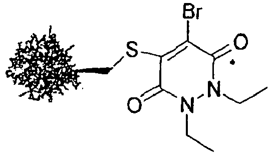

To a solution of model protein (100 µL, [Protein] 2.0 mg/mL, 100 mM sodium phosphate, 150 mM NaCl, pH 8.0) at 0°C was added DiBrDDPD (5 µL, 282 mM solution in DMF). The mixture was vortexed for 1 s then maintained at 37°C for 1 h. Analysis using LC-MS showed that the desired product had been formed in quantitative yield (mass 14427).

### Example 59: β-Mercaptoethanol-mediated Cleavage of GrB2-SH2 Domain L111C / DiBrDDPD Adduct

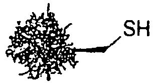

To a solution of model protein (100 µL, [Protein] 2.0 mg/mL, 100 mM sodium phosphate, 150 mM NaCl, pH 8.0) at 0°C was added DiBrDDPD (5 µL, 282 mM solution in DMF). The mixture was vortexed for 1 s then maintained at 37°C for 1 h. Analysis using LC-MS showed that the protein / DiBrDDPD adduct had been formed in quantitative yield (mass 14427).

The mixture was dialysed for 40 h at 4°C (100 mM sodium phosphate, 150 mM NaCl, pH 8.0) then treated with β-mercaptoethanol (5 µL, 2.82 M solution in H₂O) at 37°C. The mixture was vortexed for 1 s and maintained at 37°C for 2 h after which the mixture was analysed by LC-MS. Analysis showed that desired product was formed ( mass = 14180) in quantitative conversion.

### Example 60: Preparation of GrB2-SH2 Domain L111C / DiBrDDPD / β-1-thioglucose Adduct

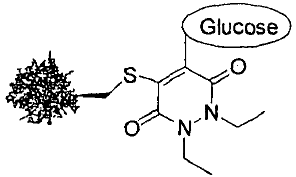

To a solution of model protein (100 µL, [Protein] 2.0 mg/mL, 100 mM sodium phosphate, 150 mM NaCl, pH 8.0) at 0°C was added DiBrDDPD (5 µL, 282 mM solution in DMF). The mixture was vortexed for 1 s then maintained at 37°C for 1 h. Analysis using LC-MS showed that the protein / DiBrDDPD adduct had been formed in quantitative yield (mass 14427).

The mixture was dialysed for 40 h at 4°C (100 mM sodium phosphate, 150 mM NaCl, pH 8.0) then treated with β-1-thioglucose (5 µL, 28.2 mM solution in H₂O) at 0°C. The mixture was vortexed for 1 s and maintained at RT for 1 h after which the mixture was analysed by LC-MS. Analysis showed that the protein / DiBrDDPD / β-1-thioglucose adduct was the only protein species present (mass = 14543).

### Example 61: β-Mercaptoethanol-mediated Cleavage of GrB2-SH2 Domain L111C / DiBrDDPD / β-1-thioglucose Adduct

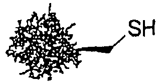

To a solution of model protein (100 µL, [Protein] 2.0 mg/mL, 100 mM sodium phosphate, 150 mM NaCl, pH 8.0) at 0°C was added DiBrDDPD (5 µL, 282 mM solution in DMF). The mixture was vortexed for 1 s then maintained at 37°C for 1 h. Analysis using LC-MS showed that the protein / DiBrDDPD adduct had been formed in quantitative yield (mass 14427).

The mixture was dialysed for 40 h at 4°C (100 mM sodium phosphate, 150 mM NaCl, pH 8.0) then treated with β-1-thioglucose (5 µL, 28.2 mM solution in H₂O) at 0°C. The mixture was vortexed for I s and maintained at RT for 1 h after which the mixture was analysed by LC-MS. Analysis showed that the protein / DiBrDDPD / β-1-thioglucose adduct was the only protein species present (mass = 14543).

The mixture was treated with β-mercaptoethanol (5 µL, 2.82 M solution in H₂O) at RT. The mixture was vortexed for 1 s and maintained at RT for 30 mins after which the mixture was analysed by LC-MS. Analysis showed that desired product was formed (mass = 14180) in quantitative conversion.

### Example 62: Preparation of GrB2-SH2 Domain L111C/BrDDPD/β-1-thioglucose Adduct

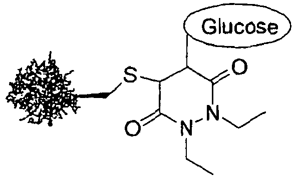

To a solution of model protein (100 µL, [Protein] 2.0 mg/mL, 100 mM sodium phosphate, 150 mM NaCl, pH 8.0) at 0°C was added BrDDPD (5 µL, 282 mM solution in DMF). The mixture was vortexed for 1 s then maintained at 37°C for 1 h. Analysis using LC-MS showed that the protein / BrDDPD adduct had been formed in quantitative yield (mass 14348).

The mixture was dialysed for 40 h at 4°C (100 mM sodium phosphate, 150 mM NaCl, pH 8.0) then treated with β-1-thioglucose (5 µL, 28.2 mM solution in H₂O) at 0°C. The mixture was vortexed for 1 s and maintained at 37°C for 1 h after which the mixture was analysed by LC-MS. Analysis showed that the protein / BrDDPD / β-1-thioglucose adduct was formed in 17% conversion (mass = 14543).

### Example 63: Preparation of GrB2-SH2 Domain L111C /Z-2,3-Dibromo-but-2-enedioic acid dimethyl ester Adduct

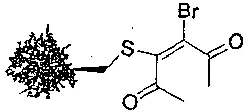

To a solution of model protein (100 µL, [Protein] 2.0 mg/mL, 100 mM sodium phosphate, 150 mM NaCl, pH 8.0) at 0°C was added Z-2,3-dibromo-but-2-enedioic acid dimethyl ester (5 µL, 282 mM solution in DMF). The mixture was vortexed for 1 s then maintained at 37°C for 1 h. Analysis using LC-MS showed that the desired product had been formed (mass 14440).

### Example 64: β-Mercaptoethanol-mediated Cleavage of GrB2-SH2 Domain L111C/ Z-2,3-Dibromo-but-2-enedioic acid dimethyl ester Adduct

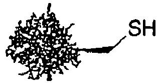

To a solution of model protein (100 µL, [Protein] 2.0 mg/mL, 100 mM sodium phosphate, 150 mM NaCl, pH 8.0) at 0°C was added Z-2,3-dibromo-but-2-enedioic acid dimethyl ester (5 µL, 282 mM solution in DMF). The mixture was vortexed for I s then maintained at 37°C for 1 h. Analysis using LC-MS showed that the desired product had been formed in quantitative yield (mass 14370).

The mixture was treated with β-mercaptoethanol (5 µL, 2.82 M solution in H₂O) at 37°C. The mixture was vortexed for 1 s and maintained at 37°C for 2 h after which the mixture was analysed by LC-MS. Analysis showed that desired product was formed (mass = 14180) in quantitative conversion.

### Example 65: Modification and regeneration of somatostatin

### Preparation of reduced somatostatin

H-Ala-Gly-Cys-Lys-Asn-Phe-Phe
SH Trp
SH Lys
HO-Cys-Ser-Thr-Phe-Thr

Lyophilised somatostatin (mass = 1638) was solubilised in buffer (50 mM sodium phosphate, pH 6.2, 40 % MeCN, 2.5 % DMF) to yield a concentration of 152.6 µM (0.25 mg/ml) and reduced with 1.1 equiv of TCEP for 1 h at ambient temperature. Completeness of the reduction was confirmed by addition of 4 equiv of dibromomaleimide to an aliquot of the sample and analysis by LC-MS.

### Bridging of Somatostatin with Halomaleimides and Derivatives

Reduced somatostatin was generated as described. 1.1 equiv of the halomaleimides or dibromomaleimide derivates (100 x stocks in 50 mM sodium phosphate, pH 6.2, 40 % MeCN, 2.5-15.0 % DMF) were added at ambient temperature and the generation of product monitored over 1 h by LC-MS. The results are shown in Figure 2.

### Bridging of somatostatin with dithiomaleimides

Reduced somatostatin was generated as described. Various amounts of dithiomaleimide (100 x stocks in 50 mM sodium phosphate, pH 6.2, 40 % MeCN, 2.5-7.5 % DMF) were added at ambient temperature and the generation of product monitored over 1 h by LC-MS. The results are shown in Figure 3.

### Modification of somatostatin with Bromomaleimide

HN
O
O
H-Ala-Gly-Cys-Lys-Asn-Phe-Phe
Trp
Lys
HO-Cys-Ser-Thr-Phe-Thr
O
HN
O

Reduced somatostatin was generated as described. 2.1 equiv of bromomaleimide (100 x stock in 50 mM sodium phosphate, pH 6.2,40 % MeCN, 7.5 % DMF) were added at ambient temperature and complete conversion to the di-addition product observed by LC-MS within 1 h.

### Cleavage of bridged somatostatin with various reducing agents

Maleimide bridged somatostatin was prepared as described. 100 equiv of various reducing agents (1000 x stock in 50 mM sodium phosphate, pH 6.2, 40 % MeCN, 2.5 % DMF) were added and the generation of unmodified peptide and side products (mixed disulfides of the reducing agents with the free peptide-cysteines) monitored at 4 °C over 2 d by LC-MS. Mixed disulfides of somatostatin with GSH could only be detected by MALDI-TOF MS. The results are shown in Figure 4.

### Cleavage of bridged somatostatin with various amounts of DTT and 2-mercaptoethanol

Maleimide bridged somatostatin was prepared as described. Various amounts of DTT or 2-mercaptoethanol (1000 x stock in 50 mM sodium phosphate, pH 6.2, 40 % MeCN, 2.5 % DMF) were added and the generation of unmodified peptide and side products (mixed disulfides of the reducing agents with the free peptide-cysteines) monitored at 4 °C over 6 h by LC-MS. The results are shown in Figure 5.

### Catalysed cleavage of bridged somatostatin

Maleimide bridged somatostatin was prepared as described. 20 equiv of 2-mercaptoethanol (1000 x stock in 50 mM sodium phosphate, pH 6.2, 40 % MeCN, 2.5 % DMF) were added followed by either buffer or 5 equiv of sodium iodide or benzeneselenol (100 x stock in 50 mM sodium phosphate, pH 6.2, 40 % MeCN, 7.5 % DMF) and the generation of unmodified peptide and side products (mixed disulfides of 2-mercaptoethanol or benzeneselenol with the free peptide-cysteines) monitored at ambient temperature over 20 min by LC-MS. The results are shown in Figure 6.

### Cleavage of N-functionalised maleimide bridged somatostatin

Somatostatin was reduced and bridged with N-functionalised maleimide derivates as described. 100 equiv of 2-mercaptoethanol (1000 x stock in 50 mM sodium phosphate, pH 6.2, 40 % MeCN, 2.5 % DMF) were added and the generation of unmodified peptide and side products (mixed disulfides of 2-mercaptoethanol with the free peptide-cysteines) monitored at 4 °C over 2 d by LC-MS. The results are shown in Figure 7.

### Cleavage of di-addition product of monobromomaleimide to somatostatin

Reduced somatostatin was reacted with 2.1 equiv of monobromomaleimide to generate the di-addition product. Next 100 equiv of 2-mercaptoethanol (1000 x stock in 50 mM sodium phosphate, pH 6.2, 40 % MeCN, 2.5 % DMF) were added and the generation of mono-addition product, unmodified peptide and side products (mixed disulfides of 2-mercaptoethanol with the free peptide-cysteines) monitored at ambient temperature over 2.5 h by LC-MS. The results are shown in Figure 8.

### Comparable in situ bridging of somatostatin

To somatostatin were added various amounts of dithiomaleimides (100 x stock in 50 mM sodium phosphate, pH 6.2, 40 % MeCN, 2.5-7.5 % DMF) and the reaction was incubated at ambient temperature for 10 min. Next various amounts of TCEP or benzeneselenol (100 x stocks, freshly prepared in 50 mM sodium phosphate, pH 6.2, 40 % MeCN, 2.5-7.5 % DMF) were added and the generation of bridged somatostatin was monitored over 1 h at ambient temperature by LC-MS. The results are shown in Figure 9.

### In situ PEGylation of somatostatin

To somatostatin were added either 5 equiv of N-PEG5000-dithiophenolmaleimide or 10 equiv of N-PEG5000-dithiophenolmaleimide (100 x stocks in 50 mM sodium phosphate, pH 6.2, 40 % MeCN, 2.5 % DMF) and the reaction was incubated at ambient temperature for 10 min. Next 3 equiv of TCEP respectively 5 equiv of benzeneselenol (100 x stocks, freshly prepared in 50 mM sodium phosphate, pH 6.2, 40 % MeCN, 2.5-7.5 % DMF) were added and the generation of PEGylated somatostatin was monitored over 2 h at ambient temperature by LC-MS. The results are shown in Figure 10.

### Modification of Somatostatin with DiBrDDPD

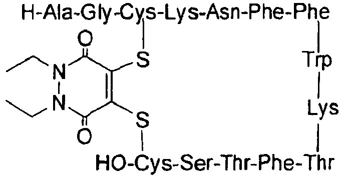
H-Ala-Gly-Cys-Lys-Asn-Phe-Phe
O
N
S
Trp
N
S
Lys
O
HO-Cys-Ser-Thr-Phe-Thr

Lyophilized somatostatin (mass = 1638) was solubilized in buffer (50 mM sodium phosphate, pH 6.2, 40% MeCN, 2.5% DMF) to yield a concentration of 152.6 µM (0.25 mg/mL) and reduced with 1.1 equiv of TCEP for 1 h at 21 °C. Completeness of the reduction was confirmed by LCMS (mass = 1640). DiBrDDPD (100 mol eq) was added and the reaction maintained at 21 °C for 10 mins. Somatostatin / DiBrDDPD adduct was observed to form quantitative conversion (mass = 1803).

### Demonstration the Retained Biological Activity of Bridged Somatostatins Using Patch-Clamping

To examine whether the bridging modification had a deleterious effect on the activity of the resultant somatostatin analogues we tested the dibromomaleimide bridged analogue , the PEGylated-dibromomaleimide bridged analogue , and the fluorescein dibromomaleimide-bridged analogue *via* a patch clamp assay. HEK 293 cells expressing HKIR3.1/3.2 channel and human somatostatin receptor 2 were treated with these compounds, and whole cell patch-clamp current recordings taken. All three analogues induced a robust activation of GIRK currents in an amplitude comparable to somatostatin iteslf. As a control when cells were treated with Pertussis toxin, or by the GIRK inhibitor Tertiapin Q, currents were largely inhibited. This data confirms that the bridged somatostatin analogues retain the biological activity of somatostatin for agonism of the somatostatin receptor 2.

### Cell culture

Cell-culture methods and the generation of stable cell lines were carried out as described in J Biol Chem 275, 921-9 (2000). HEK293 cells (human embryonic kidney cell line) stably expressing Kir3.1 and Kir3.2A channels were maintained in minimum essential medium supplemented with 10% foetal calf serum and 727 µg of G418 (Invitrogen), at 37 °C in humidified atmosphere (95% O₂, 5% CO₂). Cells were transiently transfected with SSTR2 DNA (Missouri S&T cDNA Resource Center) along with pEGFP-N1 (Clontech) for visualization of transfected cells using epifluorescence. Transfections were performed with 5 µl of Fugene HD (Roche) and 800 ng SSTR2-DNA and 40 ng EGFP-DNA per 97 µl of cell culture medium (containing no serum or antibiotics).

### Preparation of somatostatin and analogues for patch-clamp experiments

Bridged somatostatins were prepared as described above. Somatostatin and its analogues were dialysed for 24 h at 4 °C in buffer (50 mM sodium phosphate, pH 6.2) to remove the organic solvents. After dialysis the concentration was determined and the peptides stored at 4 °C. A final concentration of 20 µM somatostatin and analogues were used (dilution was done in the extracellular patch-clamp buffer).

### Electrophysiology

Whole cell patch-clamp current recordings were performed with an Axopatch 200B amplifier (Axon Instruments) using fire-polished pipettes with a resistance of 3-4 MΩ pulled from filamented borosilicated glass capillaries (Harvard Apparatus, 1.5 mm OD x 1.17 mm ID). Data was acquired and analysed via a Digidata 1322A interface (Axon Instruments) and pCLAMP software (version 8.1, Axon Instruments). A fast perfusion system was used to apply somatostatin and analogues (Rapid Solution Changer, RSC-160, Bio-Logic France). Cells were clamped at -60 mV. The extracellular solution was (mM): NaCl 80, KCl 60, CaCl₂ 2, MgCl₂ 1, HEPES 10, NaH₂PO₄ 0.33, glucose 10, pH 7.4; while the intracellular solution was (mM): K gluconate 110, KCl 20, NaCl 10, MgCl₂ 1, MgATP 2, EGTA 2 GTP 0.3, pH 7.4. After agonist application, current activated with a delay "lag" followed by a rapid rise to peak amplitude "time to peak". After removal of the agonist, the current decays back to baseline. For each cell it was assessed if flow artifacts resulting from the pressure of drug application were present. This was done by applying bath solution from one of the sewer pipes at the beginning of the recordings. Tertiapin, an inhibitor of GIRK current (Alomone), was used at a final concentration of 100 nM. Cells were incubated overnight with pertussis toxin (Sigma, 100 ng/ml), an inhibitor of Gi/o proteins. Drugs were prepared as concentrated stocks solutions and kept at -20°C.

The results are shown in Figures 11 and 12.

## Claims

1. A process, which comprises:
(i) reacting a thiol compound with a compound comprising a moiety of formula (I) 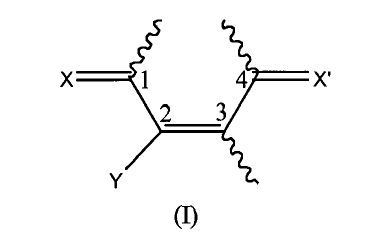 wherein X and X' are the same or different and each represents oxygen, sulfur or a group of formula =NR₁, in which R₁ is hydrogen, hydroxyl, C₁₋₆ alkyl or phenyl, and Y is an electrophilic leaving group,
thus producing a compound having a protected thiol group; and
(ii) subsequently deprotecting said protected thiol group;
and wherein at least one step is carried out after step (i) and before step (ii) which comprises effecting a chemical transformation on a functional group carried by the thiol compound that is not the protected thiol group, under conditions to which the protected thiol group is chemically inert, but to which a thiol group would be chemically reactive.

2. A process according to claim 1, wherein said compound comprising a moiety of formula (I) is a compound of formula (Ia) 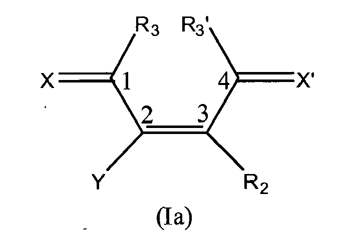 wherein:
- X and X' are the same or different and each represents oxygen, sulfur or a group of formula =NR₁, in which R₁ is hydrogen, hydroxyl, C₁₋₆ alkyl or phenyl;
- Y is an electrophilic leaving group;
- R₂ represents a hydrogen atom or a group IG;
- either:
- R₃ and R₃' are the same or different and each represents a hydrogen atom or a group IG; or
- R₃ and R₃' together form a group of formula -N(R_{33'}), wherein R_{33'} represents a hydrogen atom or a group IG; or
- R₃ and R_{3'} together form a group of formula -N(R_{33'})-N(R_{33'})-, wherein each R_{33'} is the same or different and represents a hydrogen atom or a group IG; and
- each group IG is the same or different and represents a moiety which is a C₁₋₂₀ alkyl group, a C₂₋₂₀ alkenyl group or a C₂₋₂₀ alkynyl group, which is unsubstituted or substituted by one or more halogen atoms and in which (a) 0, 1 or 2 carbon atoms are replaced by groups selected from C₆₋₁₀ arylene, 5- to 10-membered heteroarylene, C₃₋₇ carbocyclylene and 5- to 10-membered heterocyclylene groups, and (b) 0, 1 or 2 -CH₂- groups are replaced by groups selected from -O-, -S-, -S-S-, -C(O)-, -NH- and -N(C₁₋₆ alkyl)- groups, wherein:
(i) said arylene, heteroarylene, carbocyclylene and heterocyclylene groups are unsubstituted or substituted by one or more substituents selected from halogen atoms and C₁₋₆ alkyl, C₁₋₆ alkoxy, C₁₋₆ alkylthiol, -N(C₁₋₆ alkyl)(C₁₋₆ alkyl) and nitro groups; and
(ii) 0, 1 or 2 carbon atoms in said carbocyclylene and heterocyclylene groups are replaced by -C(O)- groups.

3. A process according to claim 1 or 2, wherein:
- Y is a halogen atom or a triflate, tosylate, mesylate, N-hydroxysuccinimidyl, N-hydroxysulfosuccinimidyl, C₁₋₆ alkylthiol, 5- to 10-membered heterocyclylthiol, C₆₋₁₀ arylthiol, C₃₋₇ carbocyclylthiol, -OC(O)CH₃,-OC(O)CF₃, phenyloxy, -NRₓR_{y}R_{z}⁺ or -PRₓR_{y}R_{z}⁺ group, in which Rₓ, R_{y} and R_{z} are the same or different and are selected from hydrogen atoms and C₁₋₆ alkyl and phenyl groups; or
- Y is a halogen atom or a C₁₋₆ alkylthiol, 5- to 10-membered heterocyclylthiol, C₆₋₁₀ arylthiol or C₃₋₇ carbocyclylthiol group.

4. A process according to any one of claims 1 to 3, wherein X and X' each represent oxygen.

5. A process according to any one of claims 2 to 4, wherein:
- IG represents a moiety which is an unsubstituted C₁₋₆ alkyl group, C₂₋₆ alkenyl group or C₂₋₆ alkynyl group, in which (a) 0 or 1 carbon atom is replaced by a group selected from phenylene, 5- to 6-membered heteroarylene, C₅₋₆ carbocyclylene and 5- to 6-membered heterocyclylene groups, wherein said phenylene, heteroarylene, carbocyclylene and heterocyclylene groups are unsubstituted or substituted by one or two substituents selected from halogen atoms and C₁₋₄ alkyl and C₁₋₄ alkoxy groups, and (b) 0, 1 or 2 -CH₂- groups are replaced by groups selected from -O-, -S-, -C(O)-, -NH- and -N(C₁₋₆ alkyl)-groups; or
- IG represents a moiety which is an unsubstituted C₁₋₆ alkyl group, in which (a) 0 or 1 carbon atom is replaced by a group selected from unsubstituted phenylene, 5- to 6-membered heteroarylene, C₅₋₆ carbocyclylene and 5- to 6-membered heterocyclylene groups, and (b) 0 or 1 -CH₂- groups are replaced by groups selected from -O-, -NH- and -N(C₁₋₆ alkyl)- groups; or
- IG represents an unsubstituted C₁₋₆ alkyl or phenyl group.

6. A process according to any one of claims 2 to 5, wherein R₃, R₃' and R₃₃' each represent a group IG.

7. A process according to any one of claims 2 to 6, wherein said compound of formula (Ia) is a compound of formula (Ib) 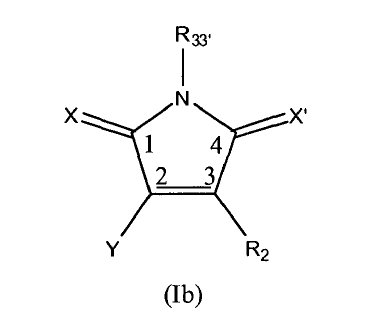 wherein X, X', Y, R₂ and R_{33'} are all as defined in any one of claims 2 to 6.

8. A process according to claim 7, wherein:
- X and X' each represent an oxygen atom;
- R_{33'} represents a hydrogen atom or a C₁₋₆ alkyl group;
- Y represents a halogen atom; and
- R₂ represents a hydrogen atom or a C₁₋₆ alkyl group.

9. A process according to any one of the preceding claims, wherein the thiol compound is cysteine or a peptide or protein comprising at least one cysteine residue.

10. A process according to any one of claims 1 to 9, wherein said step of deprotecting said protected thiol group is effected by reacting the protected thiol group with a reagent that is capable of acting as a nucleophile in a conjugate addition reaction, wherein said reagent is preferably a phosphine compound or a thiol, and wherein said reagent is more preferably tris(2-carboxyethyl)phosphine, 1,2-ethanedithiol, glutathione, 2-mercaptoethanol or dithiothreitol.

11. Use of a compound comprising a moiety of formula (I) as defined in any one of claims 1 to 8 as a reagent for protecting a thiol group in a thiol compound as defined in claim 1 or 9 during a multi-step synthesis procedure.

12. A process, which comprises:
(i) reacting a disulfide group in a disulfide compound with a compound comprising a moiety of formula (III), 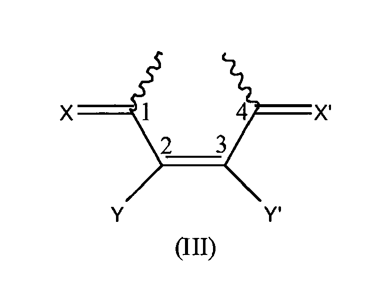 wherein X and X' are the same or different and each represents oxygen, sulfur or a group of formula =NR₁, in which R₁ is hydrogen, hydroxyl, C₁₋₆ alkyl or phenyl, and Y and Y' are the same or different and each represents an electrophilic leaving group,
thus producing a compound having a protected disulfide group; and
(ii) subsequently deprotecting said protected disulfide group;
and wherein at least one step is carried out after step (i) and before step (ii) which comprises effecting a chemical transformation on a functional group carried by the disulfide compound that is not the protected disulfide group, under conditions to which the protected disulfide group is chemically inert, but to which a disulfide group would be chemically reactive.

13. A process according to claim 12, wherein said compound comprising a moiety of formula (III) is a compound of formula (IIIa) 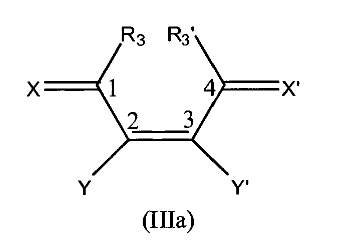 wherein X, X', Y, R₃ and R_{3'} are all as defined in any one of claims 2 to 8 and Y' is a further group Y, each group Y being the same or different.

14. Use of a compound comprising a moiety of formula (III) as defined in claim 12 or 13 as a reagent for protecting a disulfide group in a disulfide compound during a multi-step synthesis procedure.

## Patentansprüche

1. Prozess, welcher umfasst:
(i) Reagieren einer Thiolverbindung mit einer Verbindung, die eine
Komponente der Formel (I) 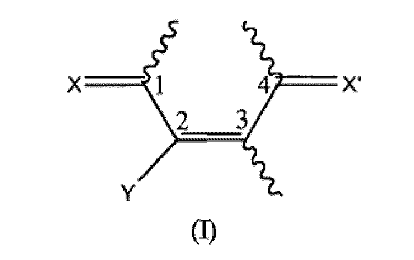 umfasst, wobei X und X' gleich oder unterschiedlich sind und jedes Sauerstoff, Schwefel oder eine Gruppe der Formel = NR₁ darstellt, wobei R₁ Wasserstoff, Hydroxyl, C₁₋₆ Alkyl oder Phenyl darstellt und Y eine elektrophile Austrittsgruppe ist,
wodurch eine Verbindung mit einer geschützten Thiolgruppe erzeugt wird; und
(ii) anschließendes Entschützen der geschützten Thiolgruppe; und wobei mindestens ein Schritt nach Schritt (i) und vor Schritt (ii) ausgeführt wird, welcher das Bewirken einer chemischen Umwandlung an einer funktionellen Gruppe, die von der Thiolverbindung getragen wird, die nicht die geschützte Thiolgruppe ist, unter Bedingungen umfasst, gegenüber denen die geschützte Thiolgruppe chemisch inert ist, aber gegenüber denen eine Thiolgruppe chemisch reaktionsbereit wäre.

2. Prozess nach Anspruch 1, wobei die eine Komponente der Formel (I) umfassende Verbindung eine Verbindung der Formel (Ia) ist: 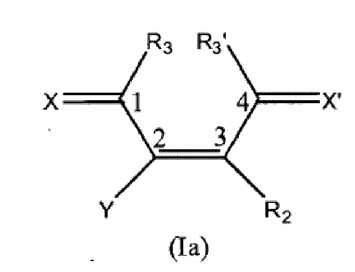 wobei:
- X und X' gleich oder unterschiedlich sind und jedes Sauerstoff, Schwefel oder eine Gruppe der Formel = NR₁ darstellt, wobei R₁ Wasserstoff, Hydroxyl, C₁₋₆ Alkyl oder Phenyl ist;
- Y eine elektrophile Austrittsgruppe ist;
- R₂ ein Wasserstoffatom oder eine Gruppe IG darstellt;
- entweder:
- R₃ und R₃' gleich oder unterschiedlich sind und jedes ein Wasserstoffatom oder eine Gruppe IG darstellt; oder
- R₃ und R₃' zusammen eine Gruppe der Formel -N(R_{33'}) darstellen, wobei R_{33'} ein Wasserstoffatom oder eine Gruppe IG darstellt; oder
- R₃ und R₃' zusammen eine Gruppe der Formel -N(R_{33'})-N(R_{33'})- bilden, wobei jedes R_{33'} gleich oder unterschiedlich ist und ein Wasserstoffatom oder eine Gruppe IG darstellt; und
- jede Gruppe IG gleich oder unterschiedlich ist und eine Komponente darstellt, die eine C₁₋₂₀ Alkylgruppe, eine C₂₋₂₀ Alkenylgruppe oder eine C₂₋₂₀ Alkynylgruppe ist, die unsubstituiert ist oder mit einem oder mehreren Halogenatomen substituiert ist, und wobei (a) 0, 1 oder 2 Kohlenstoffatome durch Gruppen gewählt aus C₆₋₁₀ Arylen, 5- bis 10-gliedriges Heteroarylen, C₃₋₇ Carbocyclylen und 5-bis-10-gliedrige Heterocyclylen-Gruppen ersetzt sind und (b) 0, 1 oder 2 -CH₂- Gruppen durch Gruppen gewählt aus -O-, -S-, -S-S-, -C(O)-, -NH- und -N(C₁₋₆ Alkyl)- Gruppen ersetzt sind, wobei:
(i) die Arylen-, Heteroarylen-, Carbocyclylen- und Heterocyclylen-Gruppen unsubstituiert sind oder mit ein oder mehreren Substituenten gewählt aus Halogenatomen und C₁₋₆ Alkyl, C₁₋₆ Alkoxy, C₁₋₆ Alkylthiol, -N(C₁₋₆ Alky)(C₁₋₆Alkyl) und Nitrogruppen substituiert; und
(ii) 0, 1 oder 2 Kohlenstoffatome in den Carbocyclylen- und Heterocyclylen-Gruppen durch -C(O)- Gruppen ersetzt sind.

3. Prozess nach Anspruch 1 oder 2, wobei:
- Y ein Halogenatom oder ein Triflat, Mesylat, N-Hydroxysuccinimidyl, N-Hydroxysulfosuccinimidyl, C₁₋₆ Alkylthiol, 5-bis-10-gliedriges Heterocyclylthiol, C₆₋₁₀Arylthiol, C₃₋₇-Carbocyclylthiol, -OC(O)CH₃,-OC(O)CF₃, Phenyloxy, -NRₓR_{y}R_{z}⁺ oder -PRₓR_{y}R_{z}⁺ Gruppe ist, wobei Rₓ, R_{y} und R_{z} gleich oder unterschiedlich sind und aus Wasserstoffatomen und C₁₋₆ Alkyl und Phenylgruppen gewählt sind; oder
- Y ein Halogenatom oder ein C₁₋₆ Alkylthiol, 5-bis-10-gliedriges Heterocyclylthiol, C₆₋₁₀ Arylthiol oder eine C₃₋₇ Carbocyclylthiolgruppe ist.

4. Prozess nach einem der Ansprüche 1 bis 3, wobei X und X' jeweils Sauerstoff darstellen.

5. Prozess nach einem der Ansprüche 2 bis 4, wobei:
- IG eine Komponente darstellt, die eine unsubstituierte C₁₋₆ Alkylgruppe, C₂₋₆ Alkenylgruppe oder C₂₋₆ Alkynylgruppe ist, wobei (a) 0 oder 1 Kohlenstofifatom durch eine Gruppe gewählt aus Phenylen, 5-bis-6-gliedrigem Heteroarylen, C₅₋₆ Carbocyclylen und 5-bis-6-gliedrigen Heterocyclylengruppen ersetzt ist, wobei die Phenylen-, Heteroarylen-, Carbocyclylen- und Heterocyclylengruppen unsubstituiert oder mit einem oder zwei Substituenten gewählt aus Halogenatomen und C₁₋₄Alkyl- und C₁₋₄ Alkoxygruppen substituiert sind und (b) 0, 1 oder 2 -CH₂- Gruppen durch Gruppen gewählt aus -O-, -S-, -C(O)-, -NH- und -N(C₁₋₆ Alkyl)-Gruppen ersetzt sind; oder
- IG eine Komponente darstellt, die eine unsubstituierte C₁₋₆ Alkylgruppe ist, wobei (a) 0 oder 1 Kohlenstofifatom durch eine Gruppe gewählt aus unsubstituiertem Phenylen, 5-bis-6-gliedrigem Heteroarylen, C₅₋₆ Carbocyclylen und 5-bis-6-gliedrigen Heterocyclylengruppen ersetzt ist und (b) 0 oder 1 -CH2- Gruppen durch Gruppen gewählt aus -O-, -NH- und -N(C₁₋₆Alkyl)- Gruppen ersetzt sind; oder
- IG eine unsubstituierte C₁₋₆ Alkyl- oder Phenyl-Gruppe darstellt.

6. Prozess nach einem der Ansprüche 2 bis 5, wobei R₃, R₃' und R₃₃' jeweils eine Gruppe IG darstellen.

7. Prozess nach einem der Ansprüche 2 bis 6, wobei die Verbindung der Formel (Ia) eine Verbindung der Formel (Ib) ist 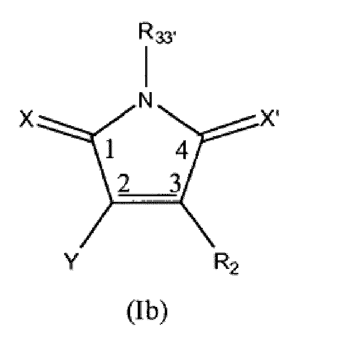 wobei X, X', Y, R₂ und R₃₃' alle wie in einem der Ansprüche 2 bis 6 festgelegt sind.

8. Prozess nach Anspruch 7, wobei:
- X und X' jeweils ein Sauerstoffatom darstellen;
- R₃₃' ein Wasserstoffatom oder eine C₁₋₆ Alkylgruppe darstellt;
- Y ein Halogenatom darstellt; und
- R₂ ein Wasserstoffatom oder eine C₁₋₆ Alkylgruppe darstellt.

9. Prozess nach einem der vorhergehenden Ansprüche, wobei die Thiolverbindung ein Cystein oder ein Peptid oder ein Protein ist, das mindestens einen Cysteinrest umfasst.

10. Prozess nach einem der Ansprüche 1 bis 9, wobei der Schritt des Entschützens der geschützten Thiolgruppe durch Reagieren der geschützten Thiolgruppe mit einem Reagens erfolgt, das in einer Konjugatzugabereaktion als Nukleophil fungieren kann, wobei das Reagens vorzugsweise eine Phosphinverbindung oder ein Thiol ist und wobei das Reagens bevorzugter tris(2-Carboxyethyl)phosphin, 1,2-Ethandithiol, Glutathion, 2-Mercaptoethanol oder Dithiothreitol ist.

11. Verwendung einer Verbindung, die eine Komponente der Formel (I) nach einem der Ansprüche 1 bis 8 umfasst, als Reagens zum Schützen einer Thiolgruppe in einer Thiolverbindung nach Anspruch 1 oder 9 während eines mehrstufigen Synthesevorgehens.

12. Prozess, welcher umfasst:
(i) Reagieren einer Disulfidgruppe in einer Disulfidverbindung mit einer Verbindung, die eine Komponente der Formel (III) umfasst: 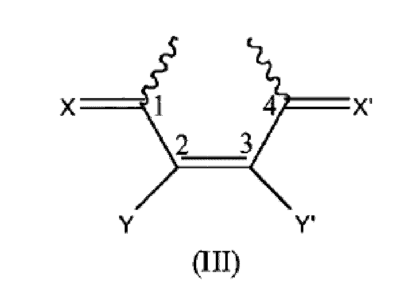 wobei X und X' gleich oder unterschiedlich sind und jedes Sauerstoff, Schwefel oder eine Gruppe der Formel = NR₁ darstellt, wobei R₁ Wasserstoff, Hydroxyl, C₁₋₆ Alkyl oder Phenyl darstellt und Y und Y' gleich oder unterschiedlich sind und jedes eine elektrophile Austrittsgruppe darstellt,
wodurch eine Verbindung mit einer geschützten Disulfidgruppe erzeugt wird; und
(ii) anschließendes Entschützen der geschützten Disulfidgruppe; und wobei mindestens ein Schritt nach Schritt (i) und vor Schritt (ii) ausgeführt wird, welcher das Bewirken einer chemischen Umwandlung an einer funktionellen Gruppe, die von der Disulfidverbindung getragen wird, die nicht die geschützte Disulfidgruppe ist, unter Bedingungen umfasst, gegenüber denen die geschützte Disulfidgruppe chemisch inert ist, aber gegenüber denen eine Disulfidgruppe chemisch reaktionsbereit wäre.

13. Prozess nach Anspruch 12, wobei die Verbindung, die eine Komponente der Formel (III) umfasst, eine Verbindung der Formel (IIIa) ist: 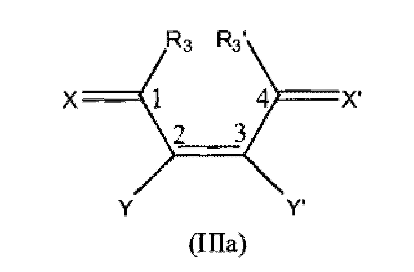 wobei X, X', Y, R₃ und R_{3'} alle wie in einem der Ansprüche 2 bis 8 festgelegt sind und Y' eine weitere Gruppe Y ist, wobei jede Gruppe Y gleich oder unterschiedlich ist.

14. Verwendung einer Verbindung, welche eine Komponente der Formel (III) nach Anspruch 12 oder 13 umfasst, als Reagens zum Schützen einer Disulfidgruppe in einer Disulfidverbindung während eines mehrstufigen Syntheseverfahrens.

## Revendications

1. Procédé qui comprend :
(i) la réaction d'un composé thiol avec un composé comprenant un fragment de formule (I) 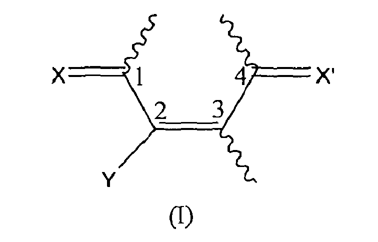 dans laquelle X et X' sont identiques ou différents et représentent chacun l'oxygène, le soufre ou un groupe de formule =NR₁ dans laquelle R₁ est l'hydrogène, un radical hydroxyle, un radical C₁₋₆-alkyle ou un radical phényle, et Y est un groupe partant électrophile,
ce qui produit ainsi un composé ayant un groupe thiol protégé ; et
(ii) ensuite la déprotection dudit groupe thiol protégé ; dans lequel au moins une étape est effectuée après l'étape (i) et avant l'étape (ii), qui comprend la mise en oeuvre d'une transformation chimique sur un groupe fonctionnel porté par le composé thiol qui n'est pas le groupe thiol protégé, dans des conditions vis-à-vis desquelles le groupe thiol protégé est chimiquement inerte, mais vis-à-vis desquelles un groupe thiol devrait être chimiquement réactif.

2. Procédé selon la revendication 1, dans lequel ledit composé comprenant un fragment de formule (I) est un composé de formule (Ia) 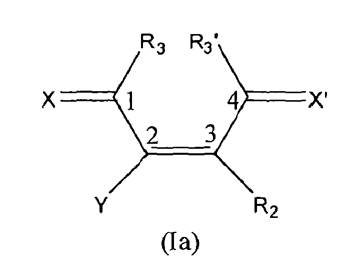 dans laquelle ;
X et X' sont identiques ou différents et représentent chacun l'oxygène, le soufre ou un groupe de formule =NR₁ dans laquelle R₁ est l'hydrogène, un radical hydroxyle, un radical C₁₋₆-alkyle ou un radical phényle ;
Y est un groupe partant électrophile ;
R₂ représente un atome d'hydrogène ou un groupe IG ;
soit R₃ et R₃' sont identiques ou différents et représentent chacun un atome d'hydrogène ou un groupe IG ;
soit R₃ et R₃' forment ensemble un groupe de formule -N(R_{33'}) dans laquelle R_{33'} représente un atome d'hydrogène ou un groupe IG ;
soit R₃ et R₃' forment ensemble un groupe de formule -N(R_{33'})-N(R_{33'})- dans laquelle chacun des R_{33'}, qui sont identiques ou différents, représente un atome d'hydrogène ou un groupe IG ;
et chacun des groupes IG, qui sont identiques ou différents, représente un fragment qui est un groupe C₁₋₂₀-alkyle, un groupe C₂₋₂₀-alcényle ou un groupe C₂₋₂₀-alcynyle, qui est non substitué ou substitué par un ou plusieurs atomes d'halogènes et dont (a) 0, 1 ou 2 atomes de carbone sont remplacés par des groupes choisis parmi les groupes C₆₋₁₀-arylène, hétéroarylène de 5 à 10 chaînons, C₃₋₇-carbocyclylène et hétérocyclylène de 5 à 10 chaînons, et (b) 0, 1 ou 2 groupes -CH₂- sont remplacés par des groupes choisis parmi les groupes -O-, -S-, -S-S-, -C(O)-, -NH- et -N(C₁₋₆-alkyle)-, où :
(i) lesdits groupes arylène, hétéroarylène, carbocyclylène et hétérocyclylène sont non substitués ou substitués par un ou plusieurs substituants choisis parmi les atomes d'halogène et les groupes C₁₋₆-alkyle, C₁₋₆-alcoxy, C₁₋₆-alkylthiol, -N(C₁₋₆-alkyle) (C₁₋₆-alkyle) et nitro ; et
(ii) 0, 1 ou 2 atomes de carbone dans lesdits groupes carbocyclylène et hétérocyclylène sont remplacés par des groupes -C(O)-.

3. Procédé selon la revendication 1 ou 2, dans lequel :
- Y est un atome d'halogène ou un groupe triflate, tosylate, mésylate, N-hydroxysuccinimidyle, N-hydroxysulfosuccinimidyle, C₁₋₆-alkylthiol, hétérocyclylthiol de 5 à 10 chaînons, C₆₋₁₀-arylthiol, C₃₋₇-carbocyclylthiol, -OC(O)CH₃, -OC(O)CF₃, phényloxy,-NRₓR_{y}R_{z}⁺ ou -PRₓR_{y}R_{z}⁺, où Rₓ, R_{y} et R_{z} sont identiques ou différents et sont choisis parmi les atomes d'hydrogène et les groupes C₁₋₆-alkyle et phényle ; ou
- Y est un atome d'halogène ou un groupe C₁₋₆-alkylthiol, hétérocyclylthiol de 5 à 10 chaînons, C₆₋₁₀-arylthiol ou C₃₋₇-carbocyclylthiol.

4. Procédé selon l'une quelconque des revendications 1 à 3, dans lequel chacun de X et X' représente l'oxygène.

5. Procédé selon l'une quelconque des revendications 2 à 4, dans lequel :
- IG représente un fragment qui est un groupe C₁₋₆-alkyle, un groupe C₂₋₆-alcényle ou un groupe C₂₋₆-alcynyle, non substitué, dont (a) 0 ou 1 atome de carbone est remplacé par un groupe choisi parmi les groupes phénylène, hétéroarylène à 5 ou 6 chaînons, C₅₋₆-carbocyclylène et hétérocyclylène à 5 ou 6 chaînons, lesdits groupes phénylène, hétéroarylène, carbocyclylène et hétérocyclylène étant non substitués ou substitués par un ou deux substituants choisis parmi les atomes d'halogène et les groupes C₁₋₄-alkyle et C₁₋₄-alcoxy, et (b) 0, 1 ou 2 groupes -CH₂- sont remplacés par des groupes choisis parmi -O-, -S-, -C(O)-, -NH- et -N- (C₁₋₆-alkyle); ou bien
- IG représente un fragment qui est un groupe C₁₋₆-alkyle non substitué, dont (a) 0 ou 1 atome de carbone est remplacé par un groupe choisi parmi les groupes phénylène non substitué, hétéroarylène à 5 ou 6 chaînons, C₅₋₆-carbocyclylène et hétérocyclylène à 5 ou 6 chaînons, et (b) 0 ou 1 groupe -CH₂- est remplacé par des groupes choisis parmi -O-, -NH- et -N- C₁₋₆-alkyle; ou bien
- IG représente un groupe C₁₋₆-alkyle ou phényle non substitué.

6. Procédé selon l'une quelconque des revendications 2 à 5, dans lequel chacun de R₃, R₃, et R_{33'} représente un groupe IG.

7. Procédé selon l'une quelconque des revendications 2 à 6, dans lequel ledit composé de formule (Ia) est un composé de formule (Ib) 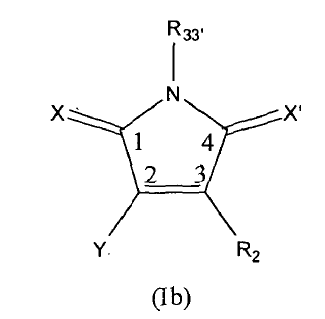 dans laquelle X, X', Y, R₂ et R_{33'} sont tous tels que définis dans l'une quelconque des revendications 2 à 6.

8. Procédé selon la revendication 7, dans lequel :
chacun de X et X' représente un atome d'oxygène ;
R_{33'} représente un atome d'hydrogène ou un groupe C₁₋₆-alkyle;
Y représente un atome d'halogène ; et
R₂ représente un atome d'hydrogène ou un groupe C₁₋₆-alkyle.

9. Procédé selon l'une quelconque des revendications précédentes, dans lequel le composé thiol est la cystéine ou un peptide ou une protéine comprenant au moins un résidu de cystéine.

10. Procédé selon l'une quelconque des revendications 1 à 9, dans lequel ladite étape de déprotection dudit groupe thiol protégé est effectuée par réaction du groupe thiol protégé avec un réactif qui est capable d'agir en tant que nucléophile dans une réaction d'addition de conjugué, ledit réactif étant de préférence un composé phosphine ou un thiol, et ledit réactif étant mieux encore la tris(2-carboxyéthyl)phosphine, le 1,2-éthanedithiol, le glutathion, le 2-mercaptoéthanol ou le dithiothréitol.

11. Utilisation d'un composé comprenant un fragment de formule (I) tel que défini dans l'une quelconque des revendications 1 à 8 en tant que réactif pour protéger un groupe thiol dans un composé thiol tel que défini dans la revendication 1 ou 9 durant une procédure de synthèse à étapes multiples.

12. Procédé qui comprend :
(i) la réaction d'un groupe disulfure dans un composé disulfure avec un composé comprenant un fragment de formule (III) 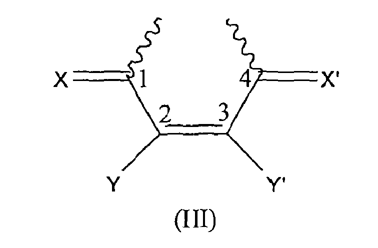 dans laquelle X et X' sont identiques ou différents et représentent chacun l'oxygène, le soufre ou un groupe de formule =NR₁ dans laquelle R₁ est l'hydrogène, un radical hydroxyle, un radical C₁₋₆-alkyle ou un radical phényle, et Y et Y' sont identiques ou différents et représentent chacun un groupe partant électrophile,
ce qui produit ainsi un composé ayant un groupe disulfure protégé ; et
(ii) ensuite la déprotection dudit groupe disulfure protégé ;
dans lequel au moins une étape est effectuée après l'étape (i) et avant l'étape (ii), qui comprend la mise en oeuvre d'une transformation chimique sur un groupe fonctionnel porté par le composé disulfure qui n'est pas le groupe disulfure protégé, dans des conditions vis-à-vis desquelles le groupe disulfure protégé est chimiquement inerte, mais vis-à-vis desquelles un groupe disulfure devrait être chimiquement réactif.

13. Procédé selon la revendication 12, dans lequel ledit composé comprenant un fragment de formule (III) est un composé de formule (IIIa) 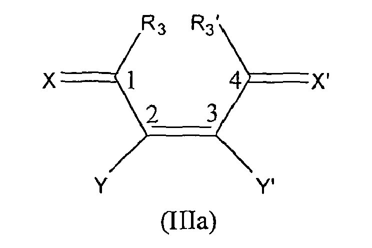 dans laquelle X, X', Y, R₃ et R₃, sont tous tels que définis dans l'une quelconque des revendications 2 à 8 et Y' est un autre groupe Y, les groupes Y étant identiques ou différents.

14. Utilisation d'un composé comprenant un fragment de formule (III) tel que défini dans la revendication 12 ou 13 en tant que réactif pour protéger un groupe disulfure dans un composé disulfure durant une procédure de synthèse à étapes multiples.
